# EUROPEAN PATENT APPLICATION

(11) **EP 4 520 821 A1**
(43) Date of publication of application: **12.03.2025**
(21) Application number: 23202952.0
(22) Date of filing: 11.10.2023
(51) Int. Cl.: C12N 15/11, A61P 35/00, C12Q 1/6883, C12N 15/113

(54) **MICRORNA-DERIVED RNAS AND POLYPEPTIDES AND USES THEREOF**

(30) Priority: 08.09.2023 US 202363581401 P
(71) Applicant: The Regents Of The University Of Michigan, Ann Arbor, Michigan 48109-1280 (US); Tianjin International Joint Academy of Biomedicine, 300457 Tianjin (CN)
(72) Inventor: CHEN, Shuang, Tianjin, 300457 (CN); WU, Haidong, Tianjin, 300457 (CN); XU, Jie, Ann Arbor, 48109 (US); HUANG, Xiaoqiang, Ann Arbor, 48109 (US); CHEN, Yuqing E., Ann Arbor, 48109 (US)
(74) Representative: HGF

(57) **Abstract**

Provided herein are microRNA-derived RNAs and polypeptides and their uses as research, diagnostic, and therapeutic agents. In particular, provided herein are systems, compositions, and methods utilizing molecules derived from templated expression of RNA via a miRNA primer.

## Description

### SEQUENCE LISTING

The text of the computer readable sequence listing filed herewith, titled "41507-731_SEQUENCE_LISTING", created 10 October 2023, is hereby incorporated by reference in its entirety.

### FIELD

Provided herein are microRNA-derived RNAs (midRs) and polypeptides (midPs) and their uses as research, diagnostic, and therapeutic agents. In particular, provided herein are systems, compositions, and methods utilizing molecules derived from templated expression of RNA via a miRNA primer.

### BACKGROUND

The discovery of new classes of biological molecules has led to significant breakthroughs in research and clinical sciences. For many years, the central dogma (DNA is used to make RNA which is used to make proteins) dominated our understanding of molecule biology. However, many important biomolecules remained undiscovered, uncharacterized, and unutilized. For example, microRNA (miRNA) was discovered in the 1990s (*See,* Lee et al., "The C. elegans heterochronic gene lin-4 encodes small RNAs with antisense complementarity to lin-14," Cell, 75 (5): 843-54 (1993)). Since its discovery, miRNA has been implicated in many important cellular functions, including gene regulation in plants and animals and has been associated with inherited diseases, cancer, heart disease, kidney disease, and obesity. The discovery and characterization of the Cas9 protein from *Streptococcus pyogenes* in 2012 led to the modern CRISPR genome editing field (See, Jinek et al., "A programmable dual-RNA-guided DNA endonuclease in adaptive bacterial immunity," Science, 337 (6096): 816-821 (2012)). Many aspects of molecular biology remain a mystery. Further elucidation of the complexities of molecular biology are needed to empower research and clinical efforts to understand and control complex biological systems. Provided herein are new classes of molecules and new molecules and their uses for research and clinical applications.

### SUMMARY

Provided herein are microRNA-derived RNAs and polypeptides and their uses as research, diagnostic, and therapeutic agents. In particular, provided herein are systems, compositions, and methods utilizing molecules derived from templated expression of RNA via a miRNA primer.

Experiments conducted during the development of the present invention discovered that the reverse pairing of miRNAs 3'-end with RNA results in a new biological effect. In this process, miRNA and the paired RNA act like "primer" and "template", leading to the production of miRNA-derived RNAs (midRs). Additional experimentation demonstrated that these midRs consequently lead to the production of miRNA-derived proteins/peptides (midPs). The newly discovered process in which midRs and midPs are produced adds a modification to the central dogma of DNA → mRNA → protein.

midRs include naturally generated molecules (e.g., RNA molecules generated from extension of an miRNA priming an RNA template) as well as synthetic versions thereof and variants thereof. midRs have a 5' region and a 3' region. The 5' region comprises an miRNA sequence and the 3' region comprises a sequence complementary to an RNA (e.g., an mRNA). For translation, midRs comprise a translational start codon and an internal ribosome entry site (IRES).

midPs include naturally generated peptides (e.g., peptides and proteins comprising an amino acid sequence encoded by an midR) as well as synthetic versions thereof (e.g., a laboratory synthesized polypeptide having an amino acid sequence corresponding to an amino acid sequence encoded by an midR) and variants thereof.

In some embodiments, provided herein are 11,453 unique human midRs. These midRs correspond to 1,293 unique midPs that are composed of 10 or more amino acids. Among them, 1,193 midPs lack any high-similarity analog in the NCBI nr database. midRs and midPs have biological functions. For example, one midP, midP0188, is a novel polypeptide that is not recorded in any database or publication. midP0188 is highly abundant in human lung and breast cancer cells. Thus, midP0188 is a novel biomarker for human lung and breast cancers. As described herein, midP0188 is linked to disease progression and its inhibition reduces tumor size and metastasis. Thus, midP0188 and the coding midRs are therapeutic targets for cancers.

In some embodiments, provided herein is an isolated miRNA-derived RNA (midR). In some embodiments, provided herein is a composition comprising an isolated miRNA-derived RNA (midR). In some embodiments, the composition consists essentially of an isolated miRNA-derived RNA (midR). In some embodiments, the midR comprises a sequence selected from the group consisting of SEQ ID NO: 8260-19712. In some embodiments, the midR consists of a sequence selected from the group consisting of SEQ ID NO: 8260-19712. In some embodiments, the midR comprises a sequence having at least 70% identity (e.g., at least 80%, 90%, 95%, 99%) to a sequence selected from the group consisting of SEQ ID NO: 8260-19712. In some embodiments, the midR encodes midP0188.

In some embodiments, provided herein is an isolated DNA comprising a sequence complementary to any of the above midRs. In some embodiments, the DNA comprises a sequence complementary to at least 50 consecutive nucleotides of any of the above midRs. In some embodiments, provided herein is a composition comprising any of the above DNAs.

In some embodiments, the midR or DNA comprises a label. In some embodiments, the label is a fluorescent label. In some embodiments, the label is one or more non-templated nucleotides.

In some embodiments, the midR or DNA is directly or indirectly attached to a solid support.

In some embodiments, the midR or DNA further comprises an adapter. In some embodiments, the midR or DNA further comprises a unique molecule identifier (UMI).

In some embodiments, the midR or DNA is fused to a nucleic acid fusion partner. In some embodiments, the fusion partner encodes a purification tag.

In some embodiments, provided herein is a composition comprising any of the midRs or DNAs above and a primer and/or probe complementary to a portion of the midR or DNA.

In some embodiments, provided herein is a composition comprising a vector comprising any of the above midRs or DNAs. In some embodiments, the vector is an expression vector.

In some embodiments, provided herein is a cell comprising a heterologous midR. In some embodiments, provided herein is a cell comprising a heterologous regulatory sequence regulating expression of a midR. In some embodiments, provided herein is a cell comprising DNA complementary to a midR. In some embodiments, the DNA is contained in a vector (e.g., an expression vector). In some embodiments, the DNA is expressible in the cell (e.g., integrated into the genome, episomally, etc.). In some embodiments, the midR is selected from the group consisting of SEQ ID NO: 8260-19712 or a variant thereof. In some embodiments, the midR encodes midP0188.

In some embodiments, provided herein is an isolated miRNA-derived peptide (midP). In some embodiments, provided herein is a composition comprising an isolated miRNA-derived peptide (midP). In some embodiments, provided herein is a composition consisting essentially of an isolated miRNA-derived peptide (midP). In some embodiments, the midP comprises a sequence selected from the group consisting of SEQ ID NO: 1-8259. In some embodiments, the midP consists of a sequence selected from the group consisting of SEQ ID NO: 1-8259. In some embodiments, the midP comprises a sequence having at least 70% identity (e.g., at least 80%, 90%, 95%) to a sequence selected from the group consisting of SEQ ID NO: 1-8259. In some embodiments, the midP or composition comprises midP0188.

In some embodiments, the midP further comprises a label. In some embodiments, the label is a fluorescent label.

In some embodiments, the midP is directly or indirectly attached to a solid support.

In some embodiment, provided herein is a composition comprising any of the above midPs and a binding molecule that specifically binds to the midP. In some embodiments, the binding molecule comprises an antibody.

In some embodiments, provided herein is a composition comprising any of the above midPs fused to a fusion partner. In some embodiments, the fusion partner is a purification tag.

In some embodiments, provided herein is a cell comprising a heterologous midP (e.g., any of the above midPs).

In some embodiments, provided herein is a cell comprising an expression vector encoding a midP (e.g., any of the above midPs). In some embodiments, the cell comprises midP0188 or an expression vector encoding midP0188.

In some embodiments, provided herein is an antibody that specifically binds to any of the above midPs.

In some embodiments, provided herein is an array comprising a solid support comprising two or more sequences complementary two or more of the above midRs. In some embodiments, the array comprises 100 or more (e.g., 200 or more, 500 or more, 1000 or more) of said sequences. In some embodiments, provided herein is an array comprising a solid support directly or indirectly attached to two or more (e.g., 20 or more, 50 or more, 100 or more, 200 or more, 500 or more, 1000 or more) of the above midRs.

In some embodiments, provided herein is a method comprising identifying the presence of one or more midR in a sample. In some embodiments, the one or more midR are selected from the group consisting of SEQ ID Nos: 8260-19712. In some embodiments, the one or more midR comprises a midR that encodes for midP0188. In some embodiments, the sample is tissue sample, a biological fluid sample, or a cancer cell (or derived from a cancer cell, etc.).

In some embodiments, provided herein is a method comprising identifying the presence of one or more midP in a sample. In some embodiments, the one or more midPs are selected from the group consisting of SEQ ID Nos: 1-8259. In some embodiments, the one or more midPs comprises midP0188. In some embodiments, the sample is tissue sample, a biological fluid sample, or a cancer cell (or derived from a cancer cell, etc.).

In some embodiments, provided herein is a method comprising depleting a cell of one or more midRs or midPs. In some embodiments, the cell resides in vitro. In some embodiments, the cell resides in vivo. In some embodiments, the method comprises introducing into the cell one or more nucleic acid sequences that bind to one or more miRNAs that generates the midRs or midPs. In some embodiments, the one or more midP comprises midP0188.

In some embodiments, provided herein is a method comprising: contacting a cell with a midR- or midP-specific inhibitor or agonist. In some embodiments, the inhibitor is an antisense oligonucleotide that binds to a midR. In some embodiments, the inhibitor is an antisense oligonucleotide that binds to an mRNA and prevents generation of a midR. In some embodiments, the inhibitor is a sequence editing system that alters the sequence of a midR. In some embodiments, the inhibitor is a sequence editing system that alters the sequence of an mRNA encoding a midR such that a functional midR is not generated from the mRNA. In some embodiments, the sequence editing system comprises a CRISPR/Cas system. In some embodiments, the midR encodes midP0188. In some embodiments, the inhibitor specifically binds to a midP. In some embodiments, the midP is midP0188. In some embodiments, the inhibitor is an antibody or antibody fragment. In some embodiments, the inhibitor is an aptamer. In some embodiments, the inhibitor prevents the generation of an miRNA. In some embodiments, the inhibitor prevents the processing of a pri-miRNA.

In some embodiments, provided herein are methods for diagnosing a disease or condition (e.g., cancer) in a subject, the method comprising the steps of: a) determining the presence of or levels of one or more midR or midP (e.g., midP0188 or midP01 12) in a sample from the subject; and b) comparing the presence of or determined levels of the midR or midP to a reference value, wherein a change in (e.g., increase or decrease in the levels of) the midR or midP in the sample is indicative of the subject having the disease or condition. In some embodiments, said midP is midP0188 and the disease is cancer.

In some embodiments, provided herein are methods for monitoring a disease or condition (e.g., cancer) in a subject, the method comprising the steps of: a) determining the levels of an midR or midP (e.g., midP0188 or midP01 12) in a first sample from the subject; and b) determining the levels of the midR or midP in a second sample from the subject, wherein the second sample has been obtained from the subject at a later time point than the first sample, wherein: i) an increase or decrease in the levels of the midR or midP in the second sample as compared to the first sample is indicative of the disease or condition progressing or regressing in the subject. In some embodiments, the midP is midP0188 and the disease is cancer.

In some embodiments, provided herein are methods for monitoring a subject's compliance to a prescribed treatment for a disease or condition (e.g., cancer), the method comprising the steps of: a) determining the levels of a midR or midP (e.g., midP0188 or midP01 12) in a first sample from the subject; and b) determining the levels of the midR or midP in a second sample from the subject, wherein the second sample has been obtained from the subject after the prescribed treatment, wherein an increase or decrease or no change in the levels of the midR or midP in the second sample as compared to the first sample is indicative of a subject's compliance to the prescribed treatment for the disease or condition. In some embodiments, the midP is midP0188 and the disease is cancer.

In some embodiments, provided herein are methods of monitoring therapeutic effect of a prescribed treatment for a disease or condition (e.g., cancer), the method comprising the steps of: a) determining the levels of an midR or midP (e.g., midP0188 or midP0112) in a first sample from the subject; and b) determining the levels of the midR or midP in a second sample from the subject, wherein the second sample has been obtained from the subject after the prescribed treatment, wherein a change or no change in the levels of the midR or midP in the second sample in the second sample as compared to the first sample is indicative of a therapeutic effect of a prescribed treatment for the disease or condition. In some embodiments, the midP is midP0188 and the disease is cancer.

In some embodiments, provided herein is a non-human animal expressing a heterologous midR. In some embodiments, the midR is a human midR. In some embodiments, the midR encodes midP0188.

In some embodiments, provided herein is a non-human animal comprising a heterologous midP. In some embodiments, the midP is a human midP. In some embodiments, the midP is midP0188.

In some embodiments, a cell comprising a midR or midP, as above, is in culture. In some embodiments, a cell comprising a midR or midP, as above, is a cancer cell. In some embodiments, a cell comprising a midR or midP, as above is a synthetic cell.

In some embodiments, provided herein is an exosome comprising a midR or midP.

In some embodiments, provided herein is a composition comprising a midP or an immunogenic fragment thereof and an adjuvant. In some embodiments, the midP is selected from the group consisting of SEQ ID NO: 1-8259. In some embodiments, the midP is midP0188.

In some embodiments, provided herein is a method of inducing an immune response, comprising: exposing an animal to an exogenous midP or an immunogenic portion thereof. In some embodiments, the exogenous midP is a synthetic midP. In some embodiments, the animal is further exposed to an adjuvant. In some embodiments, the midP is midP0188.

In some embodiments, provided herein is a method of identifying a midR or midP, comprising: a) aligning an miRNA sequence to one or more RNA sequences; b) determining a sequence of a templated extension product of said miRNA; and c) selecting extension products that contain a start codon within a defined number of bases (e.g., 100 bases) of an internal ribosome entry site (IRES). In some embodiments, the one or more or all of the aligning, determining, and selecting steps are conducted in silico. In some embodiments, the method further comprises the step of synthesizing a selected midR or midP. In some embodiments, the method further comprises the step of purifying a selected midR or midP from a sample. In some embodiments, the method further comprises the step of immunizing an animal with a selected midP or an immunogenic fragment thereof. In some embodiments, the method further comprises the step of purifying an antibody generated in the animal. In some embodiments, the method further comprises the step of contacting a sample with the antibody to identify the presence of the midP. In some embodiments, the midP is midP0188. In some embodiments, the method further comprise the step of synthesizing a primer or probe oligonucleotide that is complementary to a portion of the midR. In some embodiments, the method further comprises the step of contacting a sample with the primer or probe to identify the presence of the midR. In some embodiments, the method further comprises the step of sequencing the selected midR.

In some embodiments, provided herein is a non-transitory computer-readable medium storing a program configured to carrying out one or more steps of a) aligning an miRNA sequence to one or more RNA sequences; b) determining a sequence of a templated extension product of said miRNA; and c) selecting extension products that contain a start codon within a defined number of bases (e.g., 100 bases) of an internal ribosome entry site (IRES).

In some embodiments, provided herein is a non-transitory computer-readable medium comprising an electronic representation of one or more of SEQ ID NOs: 1-19712.

In some embodiments, provided herein is a method of sequencing at least one midR comprising: a) contacting a sample comprising one or more midRs with adapters such that one or more midR-adapter sequences are generated; and b) subjecting the midR-adapter sequences to a sequencing protocol such that at least one midR sequence read is generated. In some embodiments, the method further comprises: c) aligning the at least one midR sequence read with a reference midR sequence (e.g., a sequence known to be disease (e.g., cancer) related). In some embodiments, the adapters comprise one or more or all of the following: a universal primer binding sequence, a grafting sequence, a unique barcode, and a sample-specific index sequence.

In some embodiments, any of the above midRs or DNAs comprise at least one protecting group (e.g., phosphorothioate or 2'-O-methoxyethyl phosphorothioate).

In some embodiments, any of the above midPs comprises a protease protecting group (e.g., N-terminal or C-terminal protecting group).

### Definitions

The terms "comprise(s)," "include(s)," "having," "has," "can," "contain(s)," and variants thereof, as used herein, are intended to be open-ended transitional phrases, terms, or words that do not preclude the possibility of additional acts or structures. As used herein, comprising a certain sequence or a certain SEQ ID NO usually implies that at least one copy of said sequence is present in the recited peptide or polynucleotide. However, two or more copies are also contemplated. The singular forms "a," "and" and "the" include plural references unless the context clearly dictates otherwise. The present disclosure also contemplates other embodiments "comprising," "consisting of," and "consisting essentially of," the embodiments or elements presented herein, whether explicitly set forth or not.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9, and 7.0 are explicitly contemplated.

Unless otherwise defined herein, scientific, and technical terms used in connection with the present disclosure shall have the meanings that are commonly understood by those of ordinary skill in the art. For example, any nomenclature used in connection with, and techniques of cell and tissue culture, molecular biology, genetics and protein and nucleic acid chemistry and hybridization described herein are those that are well known and commonly used in the art. The meaning and scope of the terms should be clear; in the event, however of any latent ambiguity, definitions provided herein take precedent over any dictionary or extrinsic definition. Further, unless otherwise required by context, singular terms shall include pluralities and plural terms shall include the singular.

The terms "nucleic acid," "polynucleotide," "nucleotide sequence," and "oligonucleotide" are used interchangeably herein and refer to a polymer or oligomer of pyrimidine and/or purine bases, preferably cytosine, thymine, and uracil, and adenine and guanine, respectively (See Albert L. Lehninger, Principles of Biochemistry, at 793-800 (Worth Pub. 1982)). The terms encompass any deoxyribonucleotide, ribonucleotide, or peptide nucleic acid component, and any chemical variants thereof, such as methylated, hydroxymethylated, or glycosylated forms of these bases. The polymers or oligomers may be heterogenous or homogenous in composition, may be isolated from naturally occurring sources, or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states. In some embodiments, a nucleic acid or nucleic acid sequence comprises other kinds of nucleic acid structures such as, for instance, a DNA/RNA helix, peptide nucleic acid (PNA), morpholino nucleic acid (see, e.g., Braasch and Corey, Biochemistry, 41(14): 4503-4510 (2002) and U.S. Patent 5,034,506), locked nucleic acid (LNA; see Wahlestedt et al., Proc. Natl. Acad. Sci. U.S.A., 97; 5633-5638 (2000)), cyclohexenyl nucleic acids (see Wang, J. Am. Chem. Soc., 122; 8595-8602 (2000)), and/or a ribozyme. The terms "nucleic acid" and "nucleic acid sequence" may also encompass a chain comprising non-natural nucleotides, modified nucleotides, and/or non-nucleotide building blocks that can exhibit the same function as natural nucleotides (e.g., "nucleotide analogs"). The term encompasses sequences that include any of the known base analogs of DNA and RNA including, but not limited to, 4 acetylcytosine, 8-hydroxy-N6-methyladenosine, aziridinylcytosine, pseudoisocytosine, 5- (carboxyhydroxyl-methyl) uracil, 5 -fluorouracil, 5-bromouracil, 5-carboxymethylaminomethyl-2-thiouracil, 5-carboxymethyl-aminomethyluracil, dihydrouracil, inosine, N6-isopentenyladenine, 1 -methyladenine, 1 -methylpseudo-uracil, 1- methylguanine, 1 -methylinosine, 2,2-dimethyl-guanine, 2-methyladenine, 2-methylguanine, 3-methyl-cytosine, 5 - methylcytosine, N6-methyladenine, 7-methylguanine, 5- methylaminomethyluracil, 5-methoxy-amino-methyl-2-thiouracil, P-D-mannosylqueosine, 5'- methoxycarbonylmethyluracil, 5-methoxyuracil, 2-methylthio-N- isopentenyladenine, uracil- 5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5- oxyacetic acid methylester, uracil-5-oxyacetic acid, pseudouracil, queosine, 2-thiocytosine, and 2,6-diaminopurine.

The terms "peptide," "polypeptide," and "protein" are used interchangeably herein and refer to a polymeric form of amino acids of any length, which can include coded and non-coded amino acids, chemically or biochemically modified or derivatized amino acids, and polypeptides having modified peptide backbones. The art often refers to longer peptides as proteins (e.g., having more than 50-100 amino acids) and shorter peptides as polypeptides (e.g., having less than 50-100 amino acids). However, unless specified otherwise, the terms are used interchangeably herein to refer to polymers having at least four amino acids.

Nucleic acid or amino acid sequence "identity," as described herein, can be determined by comparing a nucleic acid or amino acid sequence of interest to a reference nucleic acid or amino acid sequence. The percent identity is the number of nucleotides or amino acid residues that are the same (e.g., that are identical) as between the sequence of interest and the reference sequence divided by the length of the longest sequence (e.g., the length of either the sequence of interest or the reference sequence, whichever is longer). A number of mathematical algorithms for obtaining the optimal alignment and calculating identity between two or more sequences are known and incorporated into a number of available software programs. Examples of such programs include CLUSTAL-W, T-Coffee, and ALIGN (for alignment of nucleic acid and amino acid sequences), BLAST programs (e.g., BLAST 2.1, BL2SEQ, and later versions thereof) and FASTA programs (e.g., FASTA3x, FAS^{™}, and SSEARCH) (for sequence alignment and sequence similarity searches). Sequence alignment algorithms also are disclosed in, for example, Altschul et al., J. Molecular Biol., 215(3): 403-410 (1990), Beigert et al., Proc. Natl. Acad. Sci. USA, 106(10): 3770-3775 (2009), Durbin et al., eds., Biological Sequence Analysis: Probabilistic Models of Proteins and Nucleic Acids, Cambridge University Press, Cambridge, UK (2009), Soding, Bioinformatics, 21(7): 951-960 (2005), Altschul et al., Nucleic Acids Res., 25(17): 3389-3402 (1997), and Gusfield, Algorithms on Strings, Trees and Sequences, Cambridge University Press, Cambridge UK (1997)).

An "oligonucleotide" refers to a nucleic acid that includes at least two nucleic acid monomer units (e.g., nucleotides), typically more than three monomer units, and more typically greater than ten monomer units. The exact size of an oligonucleotide generally depends on various factors, including the ultimate function or use of the oligonucleotide. To further illustrate, oligonucleotides are typically less than 200 residues long (e.g., between 15 and 100), however, as used herein, the term is also intended to encompass longer polynucleotide chains. Oligonucleotides are often referred to by their length. For example, a 24-residue oligonucleotide is referred to as a "24-mer". Typically, the nucleoside monomers are linked by phosphodiester bonds or analogs thereof, including phosphorothioate, phosphorodithioate, phosphoroselenoate, phosphorodiselenoate, phosphoroanilothioate, phosphoranilidate, phosphoramidate, and the like, including associated counterions, e.g., H+, NH4 +, Na+, and the like, if such counterions are present. Further, oligonucleotides are typically single-stranded. Oligonucleotides are optionally prepared by any suitable method, including, but not limited to, isolation of an existing or natural sequence, DNA replication or amplification, reverse transcription, cloning and restriction digestion of appropriate sequences, or direct chemical synthesis by a method such as the phosphotriester method of Narang et al. (1979) Meth Enzymol. 68: 90-99; the phosphodiester method of Brown et al. (1979) Meth Enzymol. 68: 109-151; the diethylphosphoramidite method of Beaucage et al. (1981) Tetrahedron Lett. 22: 1859-1862; the triester method of Matteucci et al. (1981) JAm Chem Soc. 103:3185-3191; automated synthesis methods; or the solid support method of U.S. Pat. No. 4,458,066, entitled "PROCESS FOR PREPARING POLYNUCLEOTIDES," issued Jul. 3, 1984 to Caruthers et al., or other methods known to those skilled in the art.

The term "homology" and "homologous" refers to a degree of identity. There may be partial homology or complete homology. A partially homologous sequence is one that is less than 100% identical to another sequence.

As used herein, the term "hybridization" is used in reference to the pairing of complementary nucleic acids. Hybridization and the strength of hybridization (e.g., the strength of the association between the nucleic acids) is influenced by such factors as the degree of complementary between the nucleic acids, stringency of the conditions involved, and the Tₘ of the formed hybrid. Hybridization methods involve the annealing of one nucleic acid to another, complementary nucleic acid, e.g., a nucleic acid having a complementary nucleotide sequence. The ability of two polymers of nucleic acid containing complementary sequences to find each other and "anneal" or "hybridize" through base pairing interaction is a well-recognized phenomenon. The initial observations of the "hybridization" process by Marmur and Lane, Proc. Natl. Acad. Sci. USA, 46: 453 (1960) and Doty et al., Proc. Natl. Acad. Sci. USA, 46: 461 (1960), have been followed by the refinement of this process into an essential tool of modern biology. For example, hybridization and washing conditions are now well known and exemplified in Sambrook et al., supra. The conditions of temperature and ionic strength determine the "stringency" of the hybridization.

As used herein, a "double-stranded nucleic acid" may be a portion of a nucleic acid, a region of a longer nucleic acid, or an entire nucleic acid. A "double-stranded nucleic acid" may be, e.g., without limitation, a double-stranded DNA, a double-stranded RNA, a double-stranded DNA/RNA hybrid, etc. A single-stranded nucleic acid having secondary structure (e.g., base-paired secondary structure) and/or higher order structure (e.g., a stem-loop structure) may also be considered a "double-stranded nucleic acid." For example, triplex structures are considered to be "double-stranded." In some embodiments, any base-paired nucleic acid is a "double-stranded nucleic acid."

The term "gene" refers to a DNA sequence that comprises control and coding sequences necessary for the production of an RNA having a non-coding function (e.g., a ribosomal or transfer RNA), a polypeptide, or a precursor of any of the foregoing. The RNA or polypeptide can be encoded by a full-length coding sequence or by any portion of the coding sequence so long as the desired activity or function is retained. Thus, a "gene" refers to a DNA or RNA, or portion thereof, that encodes a polypeptide or an RNA chain that has functional role to play in an organism. For the purpose of this disclosure, it may be considered that genes include regions that regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites, and locus control regions.

The terms "non-naturally occurring," "engineered," and "synthetic" are used interchangeably and indicate the involvement of the hand of humankind. The terms, when referring to nucleic acid molecules or polypeptides mean that the nucleic acid molecule or the polypeptide is at least substantially free from at least one other component with which they are naturally associated in nature and as found in nature.

A cell has been "genetically modified," "transformed," or "transfected" by exogenous nucleic acid, e.g., a vector, when such nucleic acid has been introduced inside the cell. The presence of the exogenous nucleic acid results in permanent or transient genetic change. The transforming nucleic acid may or may not be integrated (covalently linked) into the genome of the cell. For example, the transforming nucleic acid may be maintained on an episomal element such as a plasmid. With respect to eukaryotic cells, a stably transformed cell is one in which the transforming nucleic acid has become integrated into a chromosome so that it is inherited by daughter cells through chromosome replication. This stability is demonstrated by the ability of the eukaryotic cell to establish cell lines or clones that comprise a population of daughter cells containing the transforming nucleic acid. A "clone" is a population of cells derived from a single cell or common ancestor by mitosis. A "cell line" is a clone of a primary cell that is capable of stable growth in vitro for many generations.

A "subject" or "patient" may be human or non-human and may include, for example, animal strains or species used as "model systems" for research purposes, such a mouse model as described herein. Likewise, patient may include either adults or juveniles (e.g., children). Moreover, patient may mean any living organism, preferably a mammal (e.g., human or non-human) that may benefit from the administration of compositions contemplated herein. Examples of mammals include, but are not limited to, any member of the Mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. Examples of non-mammals include, but are not limited to, birds, fish, and the like. In one embodiment of the methods and compositions provided herein, the mammal is a human.

The term "contacting" as used herein refers to bring or put in contact, to be in or come into contact. The term "contact" as used herein refers to a state or condition of touching or of immediate or local proximity. Contacting a composition to a target destination, such as, but not limited to, an organ, tissue, cell, or tumor, may occur by any means of administration known to the skilled artisan.

The terms "immunogen" and "antigen" are used interchangeably herein and refer to any molecule, compound, or substance that induces an immune response in an animal (e.g., a mammal). An "immune response" can entail, for example, antibody production and/or the activation of immune effector cells. An antigen in the context of the disclosure can comprise any subunit, fragment, or epitope of any proteinaceous or non-proteinaceous (e.g., carbohydrate or lipid) molecule that provokes an immune response in a mammal. By "epitope" is meant a sequence of an antigen that is recognized by an antibody or an antigen receptor. Epitopes also are referred to in the art as "antigenic determinants." In some embodiments, an epitope is a region of an antigen that is specifically bound by an antibody. In other embodiments, an epitope may include chemically active surface groupings of molecules such as amino acids, sugar side chains, phosphoryl, or sulfonyl groups. In certain embodiments, an epitope may have specific three-dimensional structural characteristics (e.g., a "conformational" epitope) and/or specific charge characteristics. The antigen can be a protein or peptide which provokes an immune response in a mammal, preferably leading to protective immunity.

The term "binding agent," as used herein, refers to a molecule, ideally a proteinaceous molecule, which specifically binds to another molecule, such as another protein. By "antigen-binding agent" is meant a molecule, such as a proteinaceous molecule, that specifically binds to an antigen. In some embodiments, the antigen-binding agent comprises at least two components which, in combination, form the antigen-binding site of an antigen-binding agent. In some embodiments, a first component of an antigen-binding agent comprises an antibody heavy chain or a fragment thereof, and a second component of antigen-binding agent comprises an antibody light chain or fragment thereof.

The term "immunoglobulin" or "antibody," as used herein, refers to a protein that comprises at least one complementarity determining region (CDR). The CDRs form the "hypervariable region" of an antibody, which is responsible for antigen binding (discussed further below). A whole antibody typically consists of four polypeptides: two identical copies of a heavy (H) chain polypeptide and two identical copies of a light (L) chain polypeptide. Each of the heavy chains contains one N-terminal variable (VH) region and three C-terminal constant (CHI, CHI, and Cm) regions, and each light chain contains one N-terminal variable (VL) region and one C-terminal constant (CL) region. The light chains of antibodies can be assigned to one of two distinct types, either kappa (K) or lambda (X), based upon the amino acid sequences of their constant domains. In a typical antibody, each light chain is linked to a heavy chain by disulphide bonds, and the two heavy chains are linked to each other by disulphide bonds. The light chain variable region is aligned with the variable region of the heavy chain, and the light chain constant region is aligned with the first constant region of the heavy chain. The remaining constant regions of the heavy chains are aligned with each other. The variable regions of each pair of light and heavy chains form the antigen binding site of an antibody. The VH and VL regions have the same general structure, with each region comprising four framework (FW or FR) regions. The term "framework region," as used herein, refers to the relatively conserved amino acid sequences within the variable region which are located between the CDRs. There are four framework regions in each variable domain, which are designated FR1, FR2, FR3, and FR4. The framework regions form the β sheets that provide the structural framework of the variable region (see, e.g., C. A. Janeway et al. (eds.), Immunobiology, 5th Ed., Garland Publishing, New York, N.Y. (2001)). The framework regions are connected by three CDRs. As discussed above, the three CDRs, known as CDR1, CDR2, and CDR3, form the "hypervariable region" of an antibody, which is responsible for antigen binding. The CDRs form loops connecting, and in some cases comprising part of, the beta-sheet structure formed by the framework regions. While the constant regions of the light and heavy chains are not directly involved in binding of the antibody to an antigen, the constant regions can influence the orientation of the variable regions. The constant regions also exhibit various effector functions, such as participation in antibody-dependent complement-mediated lysis or antibody-dependent cellular toxicity via interactions with effector molecules and cells.

The terms "fragment of an antibody," "antibody fragment," and "antigen-binding fragment" of an antibody are used interchangeably herein to refer to one or more fragments of an antibody that retain the ability to specifically bind to an antigen (see, generally, Holliger et al., Nat. Biotech., 23(9). 1126-1129 (2005)). Any antigen-binding fragment of the antibody described herein is within the scope of the invention. The antibody fragment desirably comprises, for example, one or more CDRs, the variable region (or portions thereof), the constant region (or portions thereof), or combinations thereof. Examples of antibody fragments include, but are not limited to, (i) a Fab fragment, which is a monovalent fragment consisting of the VL, VH, CL, and CHI domains, (ii) a F(ab')2 fragment, which is a bivalent fragment comprising two Fab fragments linked by a disulfide bridge at the hinge region, (iii) a Fv fragment consisting of the VL and VH domains of a single arm of an antibody, (iv) a Fab' fragment, which results from breaking the disulfide bridge of an F(ab')2 fragment using mild reducing conditions, (v) a disulfide-stabilized Fv fragment (dsFv), and (vi) a domain antibody (dAb), which is an antibody single variable region domain (VH or VL) polypeptide that specifically binds antigen.

"Fragment," with respect to polypeptide or polynucleotide sequences, is intended a polypeptide or polynucleotide consisting of only a part of the intact full-length polypeptide or polynucleotide sequence and structure. A fragment may contain 4-100 (e.g., 5-50, 10-25, 15-20, etc.) contiguous amino acids or nucleotides found in a full-length polypeptide or polynucleotide. A fragment may contain at least 4 (e.g., 5, 6, 7, 8, 9, 10, 11, 12, 13, 14, 15, 16, 17, 18, 19, 20, ... n-1) amino acids or nucleotides found in a full-length polypeptide or polynucleotide. A polypeptide or polynucleotide "comprising" such a fragment may include additional sequences, heterologous to the full-length polypeptide or polynucleotide, that are linked to the fragment. For example, a vector comprising 50 nucleotides from a 100-nucleotide full length polynucleotide is a polynucleotide comprising the 50 nucleotide fragment in that it contains the fragment and also has nucleic acid vector sequence. Likewise, an 8 amino acid CDR derived from a first antibody and placed into a framework of a second, different antibody results in a new molecule that comprises a fragment of the first antibody.

A "portion" of an amino acid sequence comprises at least three amino acids (e.g., about 3 to about 1,200 amino acids). Preferably, a "portion" of an amino acid sequence comprises 3 or more (e.g., 5 or more, 10 or more, 15 or more, 20 or more, 25 or more, 30 or more, 40 or more, or 50 or more) amino acids, but less than 1,200 (e.g., 1,000 or less, 800 or less, 700 or less, 600 or less, 500 or less, 400 or less, 300 or less, 200 or less, or 100 or less) amino acids. Preferably, a portion of an amino acid sequence is about 3 to about 500 amino acids (e.g., about 10, 100, 200, 300, 400, or 500 amino acids), about 3 to about 300 amino acids (e.g., about 20, 50, 75, 95, 150, 175, or 200 amino acids), or about 3 to about 100 amino acids (e.g., about 15, 25, 35, 40, 45, 60, 65, 70, 80, 85, 90, 95, or 99 amino acids), or a range defined by any two of the foregoing values. More preferably, a "portion" of an amino acid sequence comprises no more than about 500 amino acids (e.g., about 3 to about 400 amino acids, about 10 to about 250 amino acids, or about 50 to about 100 amino acids, or a range defined by any two of the foregoing values).

As used herein, when an antibody or other entity (e.g., antigen binding domain) "specifically recognizes" or "specifically binds" an antigen or epitope, it preferentially recognizes the antigen in a complex mixture of proteins and/or macromolecules, and binds the antigen or epitope with affinity which is substantially higher than to other entities not displaying the antigen or epitope. In this regard, "affinity which is substantially higher" means affinity that is high enough to enable detection of an antigen or epitope which is distinguished from entities using a desired assay or measurement apparatus. Typically, it means binding affinity having a binding constant (Ka) of at least 10⁷ M⁻¹ (e.g., >10⁷ M⁻¹, >10⁸ M⁻¹, >10⁹ M⁻¹, >10¹⁰ M⁻¹, >10¹¹ M⁻¹, >10¹² M⁻¹, >10¹³ M⁻¹, etc.). In certain such embodiments, an antibody is capable of binding different antigens so long as the different antigens comprise that particular epitope. In certain instances, for example, homologous proteins from different species may comprise the same epitope.

The term "monoclonal antibody," as used herein, refers to an antibody produced by a single clone of B lymphocytes that is directed against a single epitope on an antigen. Monoclonal antibodies typically are produced using hybridoma technology, as first described in Kohler and Milstein, Eur. J. Immunol., 5; 511-519 (1976). Monoclonal antibodies may also be produced using recombinant DNA methods (see, e.g., U.S. Patent 4,816,567), isolated from phage display antibody libraries (see, e.g., Clackson et al. Nature, 352; 624-628 (1991)); and Marks et al., J. Mol. Biol., 222; 581-597 (1991)), or produced from transgenic mice carrying a fully human immunoglobulin system (see, e.g., Lonberg, Nat. Biotechnol., 23(9): 1117-25 (2005), and Lonberg, Handb. Exp. Pharmacol., 181; 69-97 (2008)). In contrast, "polyclonal" antibodies are antibodies that are secreted by different B cell lineages within an animal. Polyclonal antibodies are a collection of immunoglobulin molecules that recognize multiple epitopes on the same antigen.

A "chimeric" antibody is an antibody or fragment thereof comprising both human and non-human regions (e.g., variable regions from a mouse antibody and constant regions from a human antibody). A "humanized" antibody is a monoclonal antibody comprising a human antibody scaffold and at least one CDR obtained or derived from a non-human antibody. Non-human antibodies include antibodies isolated from any non-human animal, such as, for example, a rodent (e.g., a mouse or rat). A humanized antibody can comprise, one, two, or three CDRs obtained or derived from a non-human antibody.

The term "recombinant," as used herein, means that a particular nucleic acid (DNA or RNA) is the product of various combinations of cloning, restriction, polymerase chain reaction (PCR) and/or ligation steps resulting in a construct having a structural coding or non-coding sequence distinguishable from endogenous nucleic acids found in natural systems. DNA sequences encoding polypeptides can be assembled from cDNA fragments or from a series of synthetic oligonucleotides to provide a synthetic nucleic acid which is capable of being expressed from a recombinant transcriptional unit contained in a cell or in a cell-free transcription and translation system. Genomic DNA comprising the relevant sequences can also be used in the formation of a recombinant gene or transcriptional unit. Sequences of non-translated DNA may be present 5' or 3' from the open reading frame, where such sequences do not interfere with manipulation or expression of the coding regions and may act to modulate production of a desired product by various mechanisms. Alternatively, DNA sequences encoding RNA that is not translated may also be considered recombinant. Thus, the term "recombinant" nucleic acid also refers to a nucleic acid which is not naturally occurring, e.g., is made by the artificial combination of two otherwise separated segments of sequence through human intervention. This artificial combination is often accomplished by either chemical synthesis means, or by the artificial manipulation of isolated segments of nucleic acids, e.g., by genetic engineering techniques. Such is usually done to replace a codon with a codon encoding the same amino acid, a conservative amino acid, or a non-conservative amino acid. Alternatively, the artificial combination may be performed to join nucleic acid segments of desired functions to generate a desired combination of functions. This artificial combination is often accomplished by either chemical synthesis means, or by the artificial manipulation of isolated segments of nucleic acids, e.g., by genetic engineering techniques. When a recombinant polynucleotide encodes a polypeptide, the sequence of the encoded polypeptide can be naturally occurring ("wild type") or can be a variant (e.g., a mutant) of the naturally occurring sequence. Thus, the term "recombinant" polypeptide does not necessarily refer to a polypeptide whose sequence does not naturally occur. Instead, a "recombinant" polypeptide is encoded by a recombinant DNA sequence, but the sequence of the polypeptide can be naturally occurring ("wild type") or non-naturally occurring (e.g., a variant, a mutant, etc.). Thus, a "recombinant" polypeptide is the result of human intervention but may comprise a naturally occurring amino acid sequence.

As used herein, the term "sensitivity" is defined as a statistical measure of performance of an assay (e.g., method, test), calculated by dividing the number of true positives by the sum of the true positives and the false negatives. As used herein, the term "specificity" is defined as a statistical measure of performance of an assay (e.g., method, test), calculated by dividing the number of true negatives by the sum of true negatives and false positives.

As used herein, the term "informative" or "informativeness" refers to a quality of a marker or panel of markers, and specifically to the likelihood of finding a marker (or panel of markers) in a positive sample.

As used herein, the term "metastasis" is meant to refer to the process in which cancer cells originating in one organ or part of the body relocate to another part of the body and continue to replicate. Metastasized cells subsequently form tumors which may further metastasize. Metastasis thus refers to the spread of cancer from the part of the body where it originally occurs to other parts of the body.

The term "neoplasm" as used herein refers to any new and abnormal growth of tissue. Thus, a neoplasm can be a non-malignant neoplasm, a premalignant neoplasm or a malignant neoplasm. The term "neoplasm-specific marker" refers to any biological material that can be used to indicate the presence of a neoplasm. Examples of biological materials include, without limitation, nucleic acids, polypeptides, carbohydrates, fatty acids, cellular components (e.g., cell membranes and mitochondria), and whole cells.

By "isolated" is meant, when referring to a polypeptide, that the indicated molecule is separate and discrete from the whole organism with which the molecule is found in nature or is present in the substantial absence of other biological macro-molecules of the same type. The term "isolated" with respect to a polynucleotide is a nucleic acid molecule devoid, in whole or part, of sequences normally associated with it in nature; or a sequence, as it exists in nature, but having heterologous sequences in association therewith; or a molecule disassociated from the chromosome.

As used herein, the term "heterologous" refers to a nucleic acid or polypeptide that is not in its natural environment. For example, a heterologous gene includes a gene from one species introduced into another species. A heterologous gene also includes a gene native to an organism that has been altered in some way (e.g., mutated, added in multiple copies, linked to non-native regulatory sequences, etc.). Heterologous genes are distinguished from endogenous genes in that the heterologous gene sequences are typically joined to DNA sequences that are not found naturally associated with the gene sequences in the chromosome or are associated with portions of the chromosome not found in nature (e.g., genes expressed in loci where the gene is not normally expressed). A heterologous polypeptide includes a polypeptide from one species introduced into another species. For example, a mammalian polypeptide may be expressed in a bacterial (e.g., *E. coli*) host cell and is said to be "heterologous" when present in the non-native host cell.

As used herein, the term "label" refers to a detectable moiety that may be directly or indirectly attached to another molecule to permit detection of that other molecule. In some embodiments, the label the is a fluorophore. Examples of fluorophores include: 7-amino-4-methylcoumarin-3-acetic acid (AMCA); 5-(and -6)-carboxy-X-rhodamine, lissamine rhodamine B, 5-(and -6)-carboxyfluorescein; fluorescein-5-isothiocyanate (FITC); 7-diethylaminocoumarin-3-carboxylic acid, tetramethyl-rhodamine-5-(and -6)-isothiocyanate; 5-(and -6)-carboxytetramethylrhodamine; 7-hydroxy-coumarin-3-carboxylic acid; 6-[fluorescein 5-(and -6)-carboxamido]hexanoic acid; N-(4,4-difluoro-5,7-dimethyl-4-bora-3a, 4a diaza-3-indacenepropionic acid; eosin-5-isothiocyanate; erythrosine-5-isothiocyanate; 5-(and -6)-carboxyrhodamine 6G; and Cascades blue aectylazide (Molecular Probes, Inc., Eugene, Oreg.). In some embodiments, the fluorophore is combined with a quencher moiety to provide a FRET detection system. In some embodiments, the label is a luminescent moiety. In some embodiments, the label is a radioisotope, chromophore, mass label, electron dense particle, magnetic particle, spin label, electrochemically active molecule, enzyme, cofactor, and/or enzyme substrate. Luminescent agents include, for example, radioluminescent, chemiluminescent, bioluminescent, and phosphorescent label containing moieties.

As used herein, "CRISPR-Cas system" refers collectively to components sufficient to effect nucleic acid modification via a CRISPR/Cas mechanism. In bacteria and archaea, CRISPR/Cas systems provide immunity by incorporating fragments of invading phage, virus, and plasmid DNA into CRISPR loci and using corresponding CRISPR RNAs ("crRNAs") to guide the degradation of homologous sequences. Transcription of a CRISPR locus produces a "pre-crRNA," which is processed to yield crRNAs containing spacer-repeat fragments that guide effector nuclease complexes to cleave dsDNA sequences complementary to the spacer. Several different types of CRISPR systems are known (e.g., type I, type II, or type III), and classified based on the Cas protein type and the use of a proto-spacer-adjacent motif (PAM) for selection of proto-spacers in invading DNA. Engineering CRISPR/Cas systems for use in eukaryotic cells typically involves reconstitution of the CRISPR/Cas complex. Typically, the RNA sequences necessary for CRISPR/Cas systems are referred to collectively as "guide RNA" (gRNA) or single guide RNA (sgRNA). Thus, the terms "guide RNA," "single guide RNA," and "synthetic guide RNA," are used interchangeably herein and may refer to a nucleic acid sequence comprising a tracrRNA and a pre-crRNA array containing a guide sequence. The terms "guide sequence," "guide," and "spacer," are used interchangeably herein and refer to the nucleotide sequence within a guide RNA that specifies the target site. An engineered CRISPR-Cas system may be derived from a CRISPR-Cas system of any type or subtype. In some embodiments, the engineered CRISPR-Cas system is derived from a Type I CRISPR-Cas system. Type I system is the most widespread and diversified type of CRISPR and is further classified into subtypes.

As used herein, the phrase "solid support" refers to any surface to which biomolecules may be directly or indirectly attached or associated. Exemplary solid supports include, but are not limited to, surfaces provided by microarrays and wells, particles such as beads, columns, optical fibers, wipes, glass and modified or functionalized glass, quartz, mica, diazotized membranes (paper or nylon), polyformaldehyde, cellulose, cellulose acetate, paper, ceramics, metals, metalloids, semiconductive materials, quantum dots, coated beads or particles, other chromatographic materials, magnetic particles; plastics (including acrylics, polystyrene, copolymers of styrene or other materials, polypropylene, polyethylene, polybutylene, polyurethanes, TEFLO, etc.), polysaccharides, nylon or nitrocellulose, resins, silica or silica-based materials including silicon and modified silicon, carbon, metals, inorganic glasses, plastics, ceramics, conducting polymers (including polymers such as polypyrrole and polyindole); micro or nanostructured surfaces such as nucleic acid tiling arrays, nanotubes, nanowires, or nanoparticulate-decorated surfaces; or porous surfaces or gels such as methacrylates, acrylamides, sugar polymers, cellulose, silicates, or other fibrous or stranded polymers. In some embodiments, the solid support is a planar surface or a particle, such as a bead or microsphere. The bead can be a polystyrene, brominated polystyrene, polyacrylic acid, polyacrylonitrile, polyacrylamide, polyacrolein, polydimethylsiloxane, polybutadiene, polyisoprene, polyurethane, polyvinyl acetate, polyvinylchloride, polyvinylpyridine, polyvinylbenzylchloride, polyvinyltoluene, polyvinylidene chloride, polydivinylbenzene, polyglycidylmethacrylate, polymethylmethacrylate, or copolymers, blends, composites, or combination thereof.

The term "sample" is used in its broadest sense. In one sense it can refer to an animal cell or tissue. In another sense, it refers to a specimen or culture obtained from any source, as well as biological and environmental samples. Biological samples may include materials taken from a patient including, but not limited to cultures, blood, plasma, serum, saliva, cerebral spinal fluid, pleural fluid, milk, lymph, sputum, semen, needle aspirates, and the like. Biological samples may be obtained from all of the various families of domestic animals, as well as feral or wild animals, including, but not limited to, such animals as ungulates, bear, fish, rodents, etc. Biological samples may be cell-free samples. Environmental samples include environmental material such as surface matter, soil, water and industrial samples, as well as samples obtained from food and dairy processing instruments, apparatus, equipment, utensils, disposable and non-disposable items. These examples are not to be construed as limiting the sample types applicable to the present invention.

As used herein, the terms "processor" and "central processing unit" or "CPU" are used interchangeably and refer to a device that is able to read a program from a computer memory (e.g., ROM or other computer memory) and perform a set of steps according to the program.

As used herein, the terms "computer memory" and "computer memory device" refer to any storage media readable by a computer processor. Examples of computer memory include, but are not limited to, RAM, ROM, computer chips, digital video discs (DVD), compact discs (CDs), hard disk drives (HDD), and magnetic tape.

As used herein, the term "computer readable medium" refers to any device or system for storing and providing information (e.g., data and instructions) to a computer processor. Examples of computer readable media include, but are not limited to, DVDs, CDs, hard disk drives, magnetic tape and servers for streaming media over networks. The computer readable medium may be non-transitory or include a device of system that is not a transitory signal.

As used herein, the term "in electronic communication" refers to electrical devices (e.g., computers, processors, etc.) that are configured to communicate with one another through direct or indirect signaling.

### Description of Figures

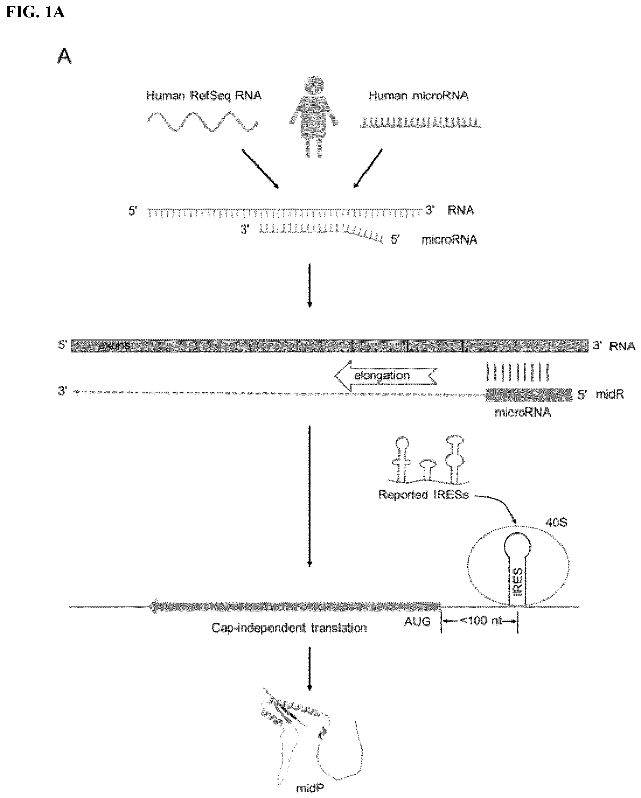

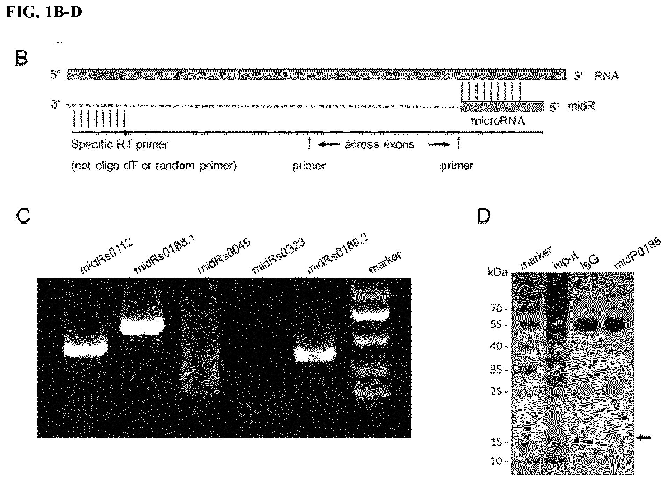

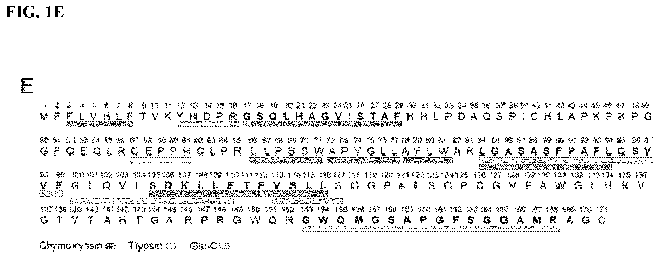

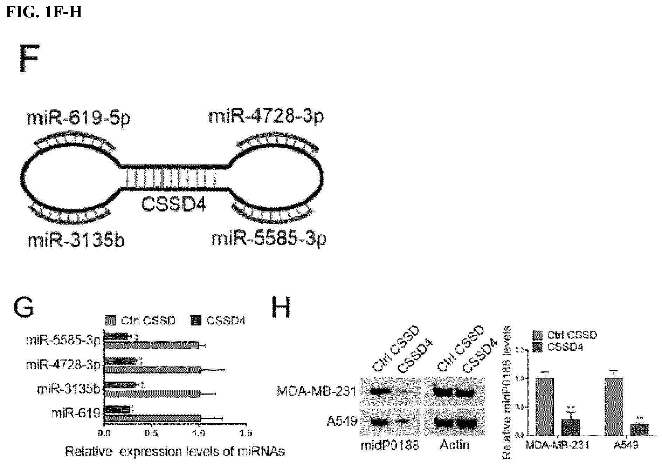

FIG. 1 shows a prediction and validation methodology for miRNA-derived RNAs (midRs) and polypeptides (midPs). FIG. 1A: Bioinformatic workflow to predict midRs and midPs. FIG. 1B: Scheme for midR specific reverse transcription (RT) and amplification primer design. FIG. 1C: Agarose gel electrophoresis analysis of midR fragments amplified by specific primers. Two regions were analyzed for midRs that encode midP0188. FIG. 1D: Silver staining of proteins captured by midP0188 antibody after SDS-PAGE electrophoresis separation. FIG. 1E: Mass spectrometry analysis to confirm midP0188 after digestion using trypsin, chymotrypsin, and Clu-C. FIG. 1F: Schematic diagram of CSSD4 simultaneously adsorbing 4 miRNAs (miR-619-5p, miR-5585-3p, miR-4728-3p, and miR-3135b) with more than 12 bp of base pairs of AP1M1 mRNA. FIG. 1G: PCR-determined expression levels of miR-619-5p, miR-5585-3p, miR-4728-3p, and miR-3135b in A549 cells after CSSD4 transfection in A549 cells. FIG. 1H: midP0188 expression levels in A549 and MDA-MB-231 cells after CSSD4 transfection.

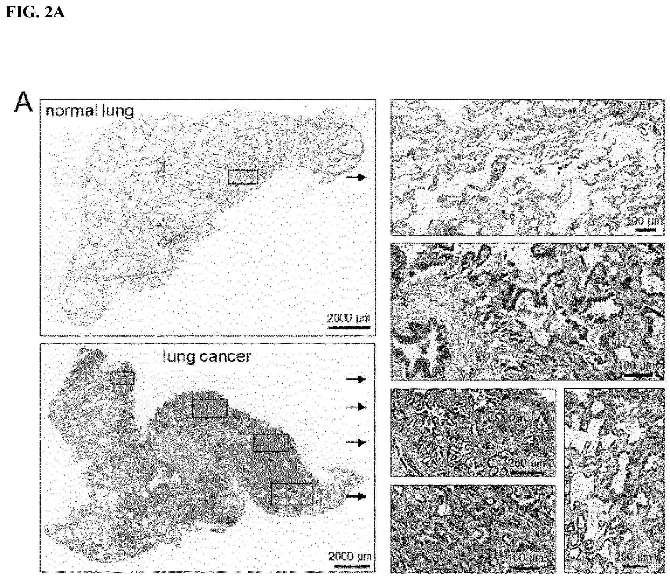

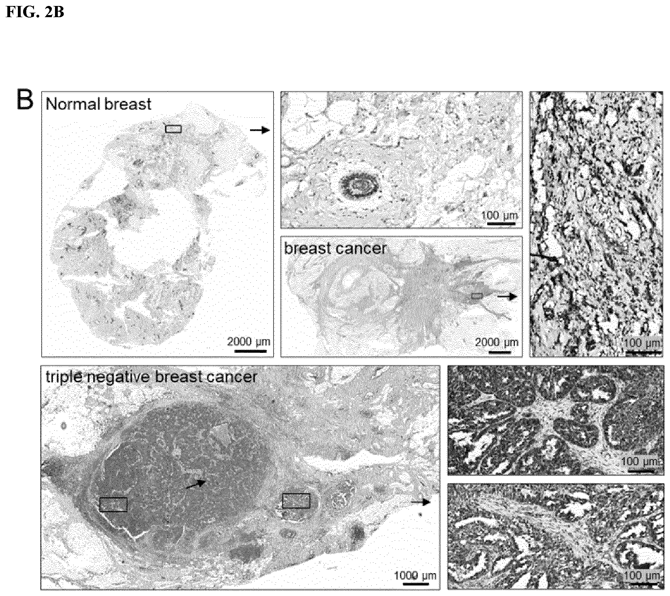

FIG.2 shows high expression of midP0188 in lung cancer and breast cancer. (A) Immunohistochemical staining results with midP0188 antibody in normal lung and lung cancer tissues. (B) Staining intensity of midP0188 in normal breast, general breast cancer and triple negative breast cancer.

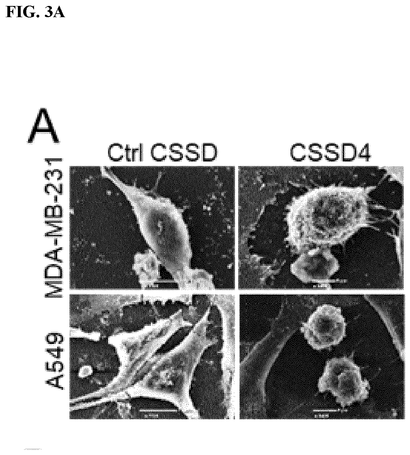

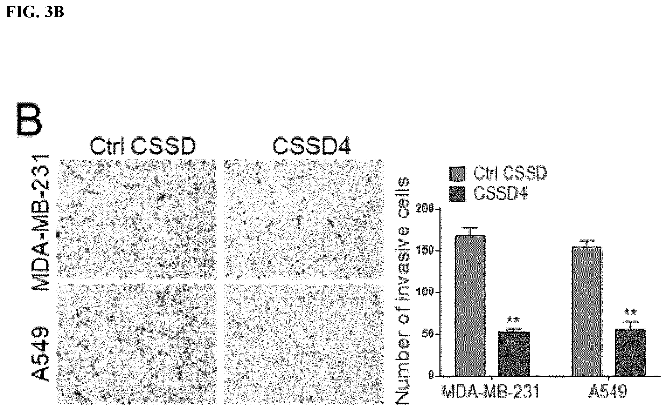

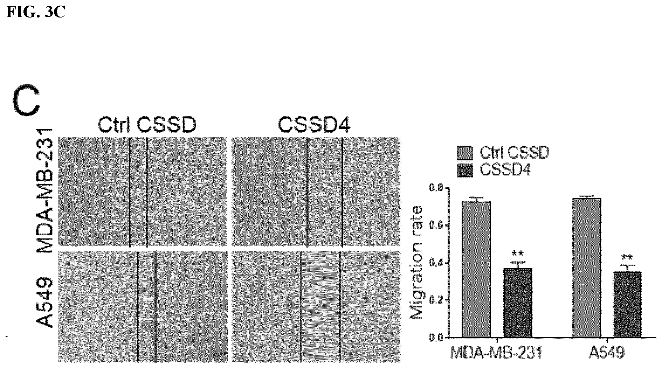

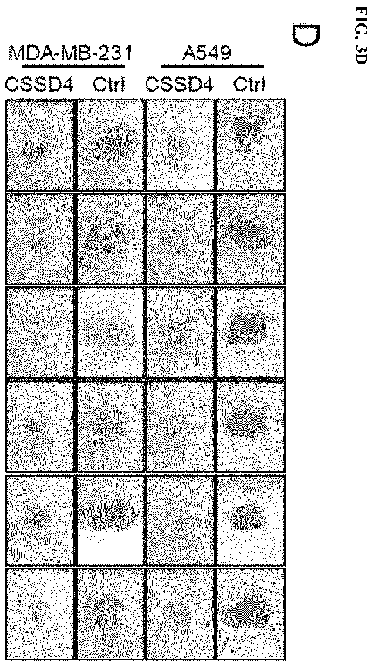

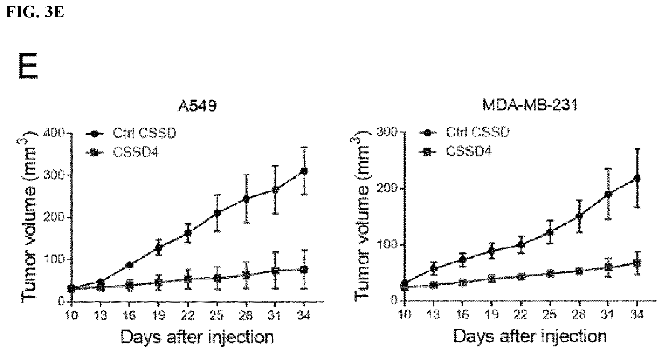

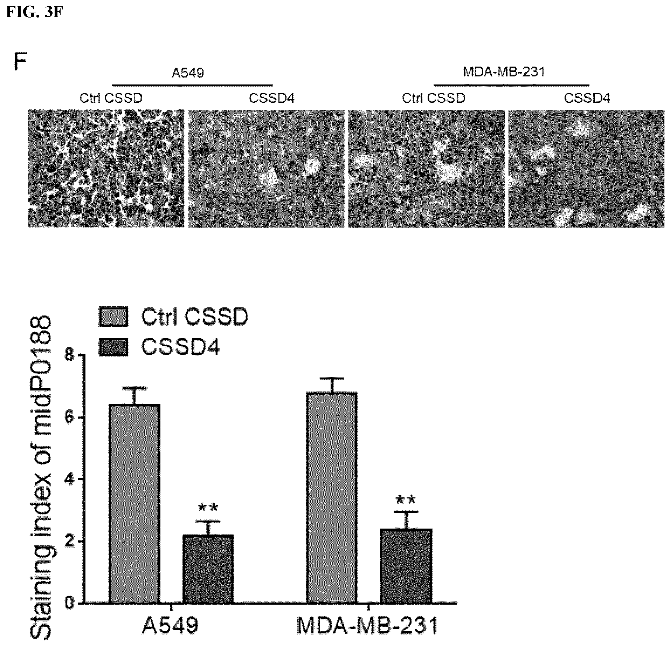

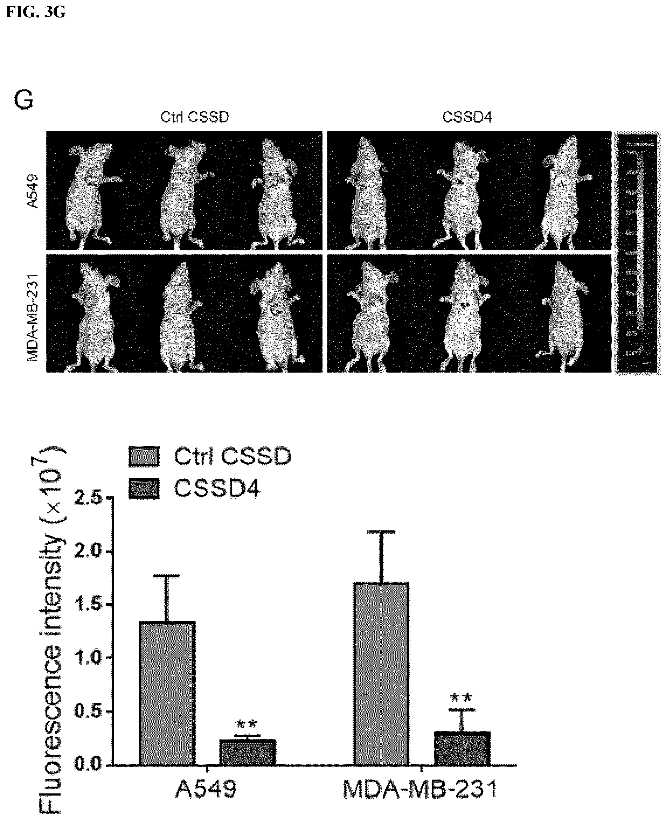

FIG 3 shows that downregulation of midP0188 reduces the invasion and migration of lung and breast cancer cells in vitro and reduces lung and breast tumor genesis and metastasis in vivo. FIG. 3A: Imaging of cell phenotype by scanning electron microscopy. FIG. 3B: Effect of CSSD4 on cell invasion by transwell assay in A549 and MDA-MB-231 cells. FIG. 3C: Effect of CSSD4 on cell migration rate by wound healing assay in A549 and MDA-MB-231 cells. FIG. 3D: CSSD4 treatment reduces the sizes of solid tumors of A549 and MDA-MB-231 cells in nude mice. FIG. 3E: Progression of the volume of solid tumors (in Fig. 3D) with time. FIG. 3F: Immunohistochemical staining of midP0188 in solid tumors. FIG. 3G: Lung metastasis of A549 and MDA-MB-231 cells.

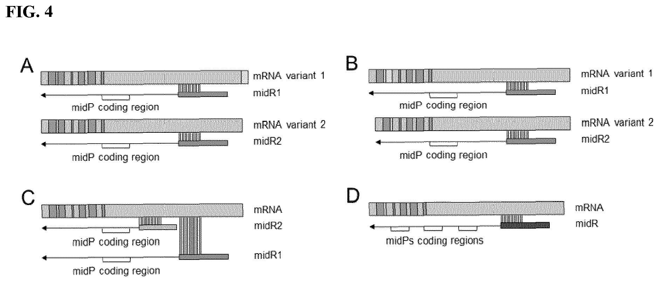

FIG. 4 shows scenarios for midR and midP derivation. FIG. 4A: RNA splicing beyond the miRNA elongation region yields identical midRs (midR1 = midR2). The excised exon in mRNA variant 1 is shown in cyan. FIG. 4B: RNA splicing within the miRNA elongation region yields different midRs (midR1 ≠ midR2) which can encode identical midPs. FIG. 4C: Different miRNAs bound to the same RNA to derive different midRs (midR1 ≠ midR2) which can encode identical midPs. FIG. 4D: One midR may encode multiple midPs.

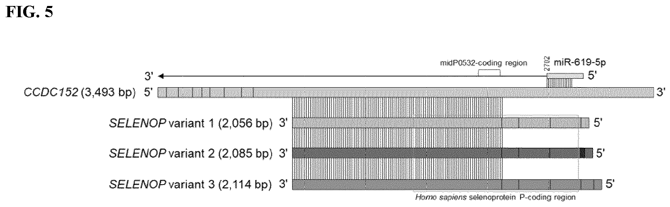

FIG. 5 shows derivation of midP0532 and related midR based on miR-619-5p and mRNA CCDC152. CCDC152 has a 1,453 bp-long reverse complementarity to all three SELENOP variants that can encode the protein, selenoprotein P. midP0532 is the same as the selenoprotein P, plasma, 1 encoded by SEPP1 (a truncated coding region of SELENOP) through conceptual translation. The exons on each mRNA are shown as rectangle blocks.

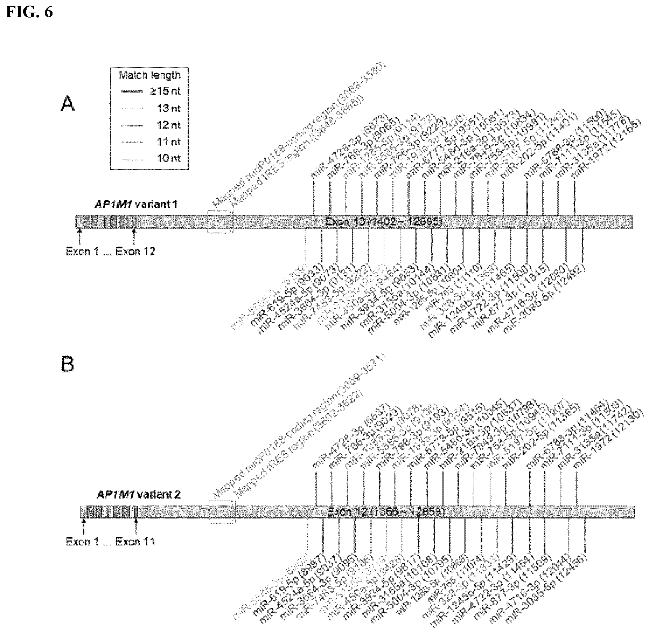

FIG. 6 shows miRNAs bound to the AP1M1 mRNA with 10- to 15-nt match. FIG. 6A: For variant 1, all ≥10-nt matches are shown after position 9,033, and only ≥12-nt matches are shown before position 9,033. FIG. 6B: For variant 2, all ≥10-nt matches are shown after position 8,997, and only ≥12-nt matches are shown before position 8,997.

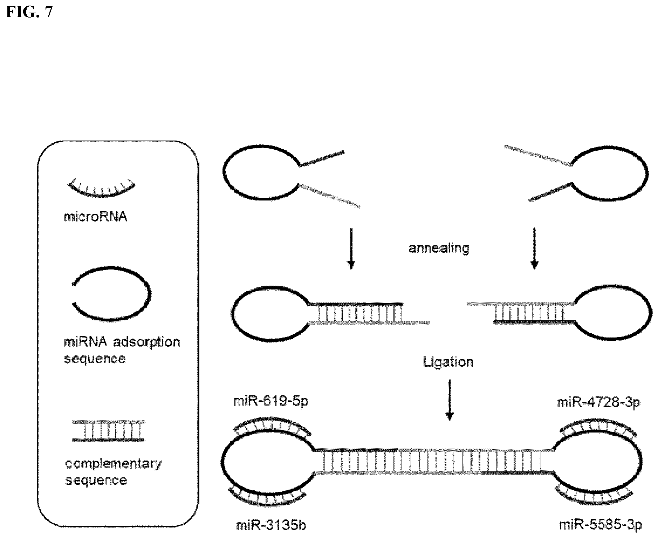

FIG. 7 shows a diagram of a route to design CSSD4. CSSD4 is designed to bind midR-619-5p, miR-3135b, miR-4728-3p, and miR-5585-3p simultaneously.

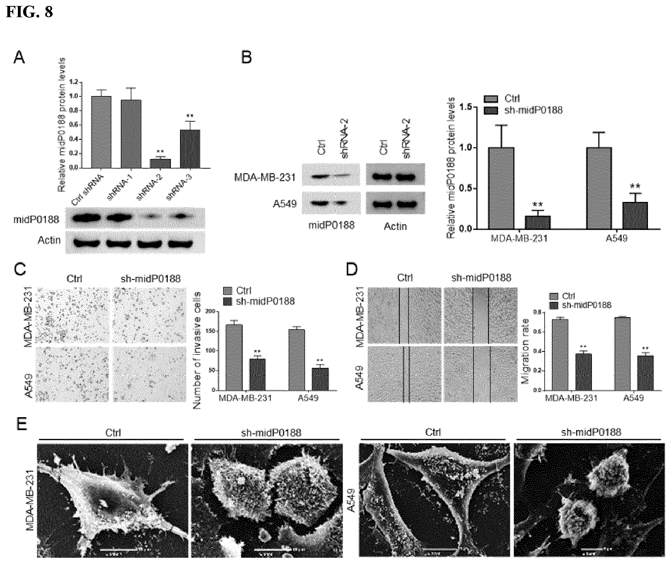

FIG. 8 shows downregulation of midP0188 by transfection of shRNAs inhibits the progression of lung and breast cancer cells in vitro. FIG. 8A: Efficacy comparison of four shRNAs. FIG. 8B: Downregulation of midP0188 levels in cancer cells. FIG. 8C: Effects of sh-midP0188 on the invasion of A549 and MDA-MB-231 cells. FIG. 8D: Effects of sh-midP0188 on the migration of A549 and MDA-MB-231 cells. FIG. 8E: Observation of cell phenotype by scanning electron microscopy after sh-midP0188 transfection. A549, lung cancer cell line. MDA-MB-231, breast cancer cell line.

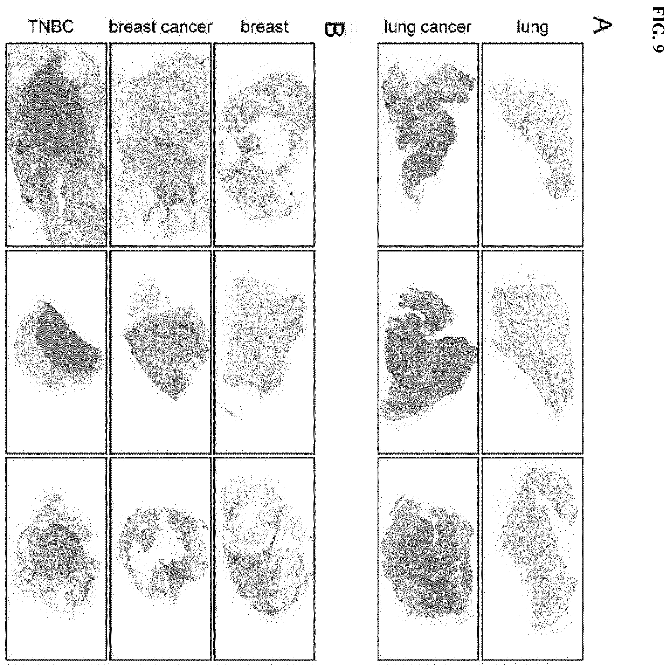

FIG. 9 shows immunohistochemistry staining for tissues from different patients using the anti-midP0188 polyclonal antibody. (A) midP0188 staining full section images for three normal lungs and three lung cancer tissues. (B) midP0188 staining full section Images for three normal breast tissues, three breast cancer tissues, and three triple-negative breast cancer (TNBC) tissues. Each image is from a different patient.

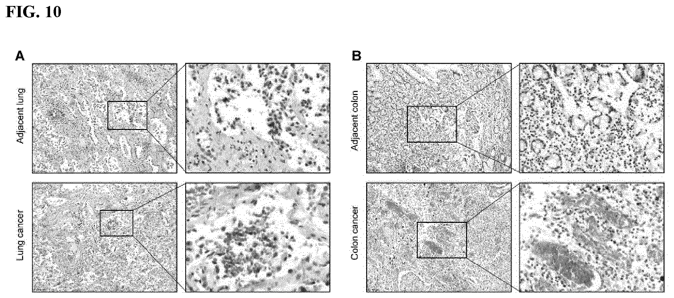

FIG. 10 shows expression ofmidP0112 in lung and colon cancer tissues. (A) Immunohistochemical staining intensity with the anti-midP0112 polyclonal antibody in lung cancer and adjacent lung tissues (left). Sections bounded by black rectangles are magnified to 100x (right) for each core section. (B) The staining intensity with the anti-midP0112 polyclonal antibody in colon cancer and adjacent colon specimens (left). Sections bounded by black rectangles are zoomed in by 100x (right) for each core section.

### DETAILED DESCRIPTION

MicroRNAs (miRNAs), are a class of short noncoding RNAs that can regulate gene expression at the post-transcriptional level.¹ Since the first miRNA (cel-lin-4) discovered in *C*. *elegans,* accumulating evidence confirms the important role of miRNAs in diseases such as cancer.²⁻⁶ Existing studies on miRNAs mainly focus on their roles in post-transcriptional regulation of gene expression, based on reverse binding of the miRNA 5'-end to mRNAs.⁷⁻⁹

During the development of embodiments of the present invention, a bioinformatics workflow was developed to predict miRNA-derived RNAs (midRs) and proteins (midPs) (FIG. 1A). For each human miRNA, its 3'-end (e.g., 15 nt) was searched against the NCBI RefSeq_RNA database to identify human RNAs with full reverse complementarity (i.e., 15-nt match). The matched miRNA and RNA were then used as "primer" and "template" respectively to generate midR, through elongation from the matched position through the RNA 5'-end. Two criteria were considered when translating a midR: (1) it contains at least an internal ribosome entry site (IRES) that allows for translation initiation in a cap-independent manner; (2) a start codon is located nearby downstream the IRES (e.g., <100 nt). Since there is currently no effective method to accurately determine IRES, the presence of an IRES was predicted by aligning midR with experimental IRESs, and assuming that a potential IRES exists in a midR if both coverage and aligned identity are sufficiently high (see Examples, below). Once an IRES is found, start codons are examined. A midR may contain multiple IRESs and following an IRES may exist multiple start codons. All possibilities are considered when translating midRs. Starting from 2,656 human miRNAs, 11,453 (SEQ ID Nos: 8260-19,712) unique midRs and 1,239 unique midPs were derived using the above parameters after removing midPs with <10 amino acids. These midPs are provided in SEQ ID Nos: 1-1239. Additional midPs were identified by setting the miRNA 3' end match to 14 nt (SEQ ID Nos: 1240-2154), 13 nt (SEQ ID Nos: 2155-4125), 12 nt (SEQ ID Nos: 4126-5976), 11 nt (SEQ ID Nos: 5977-7527), and 10 nt (SEQ ID Nos: 7528-8259), respectively.

The following sequence ID numbers are disclosed in the application:

| **SEQ ID NO:** | **Note** | **Molecule type** | **mol_type** | **Organism** |
|---|---|---|---|---|
| 1 to 8259 | midP | AA | Protein | *Homo sapiens* |
| 45 and 19713 | midP0045 | AA | Protein | *Homo sapiens* |
| 112 and 19714 | mid P0112 | AA | Protein | *Homo sapiens* |
| 188 and 19715 | midP0188 | AA | Protein | *Homo sapiens* |
| 323 and 19716 | midP0323 | AA | Protein | *Homo sapiens* |
| 19717 | shRNA-1 | RNA | Other RNA | Synthetic construct |
| 19718 | shRNA-2 | RNA | Other RNA | Synthetic construct |
| 19719 | shRNA-3 | RNA | Other RNA | Synthetic construct |
| 19720 | ctrl-shRNA | RNA | Other RNA | Synthetic construct |
| 19721 to 19723 | midRs0045 primers | DNA | Other DNA | Synthetic construct |
| 19724 to 19726 | midRs0112 primers | DNA | Other DNA | Synthetic construct |
| 19727 to 19729 | midRs0188.1 primers | DNA | Other DNA | Synthetic construct |
| 19730 to 19732 | midRs0188.2 primers | DNA | Other DNA | Synthetic construct |
| 19733 to 19735 | ssmidRs0323 primers | DNA | Other DNA | Synthetic construct |
| 19736 to 19738 | CSSD | DNA | Other DNA | Synthetic construct |

SEQ ID NO's 8259 to 19712 are a new type of RNA molecule. SEQ ID NO's 8259 to 19712 are midRs which can be either natural or synthetic RNA.

miRNAs, midRs and midPs do not always have a one-to-one correspondence relationship. Multiple IRESs on one midR may give rise to different midPs. Different miRNAs may bind to the same RNA to result in different midRs but possibly the same midP. In addition, the same miRNA may bind to the same sequences on different RNA variants (e.g., different RNA splicing variants) therefore leading to the same resultant midR and midP.

Among the 1,239 midPs identified using the 15 nt cut-off, only 46 have a high-similarity sequence analog in the NCBI nr protein database with high coverage and aligned identity. Of them, six midPs have exact sequence matches in the nr database. Of note, the 100-aa midP0532 is the same as the selenoprotein P, plasma, 1 encoded by SEPP1 (a truncated coding sequence of SELENOP) through conceptual translation (Science 291 (5507), 1304-1351 (2001)). According to the analysis, midP0532 is encoded by the midR (with identifier midR.hsa-miR-619-Sp.1519242743(CCDC152).2702) that was generated by the elongation of miRNA hsa-miR-619-5p bound to CCDC152 (homo sapiens coiled-coil domain containing 152; a 3,493-bp mRNA) at position 2702; CCDC152 has a 1,453 bp-long reverse complementarity to SELENOP (FIG. 5). CCDC152 was recently reported as an endogenous long non-coding RNA that inhibits selenoprotein P translation (Nucleic Acids Research, 2021, Vol. 49, No. 12 6893-6907).

The remaining 1,193 midPs (96%, 1,193/1,239) midPs do not have a high-similarity sequence analog in the nr database, representing a brand-new cohort of peptides from human cells.

The 1,193 midPs were compared with the peptide mass spectra (MS) profiles of multiple tumor cell lines that were previously characterized. It was revealed that 941 midPs (79% of total) have corresponding peaks in the MS data, suggesting their existence in human tumor cells.

### midP and midR identification and discovery

In some embodiments, provided herein are compositions, kits, systems, and methods that find use for the identification and discovery of midPs and midRs. midPs and midRs may be identified from any organism, tissue, cell, or mixture that contains miRNAs. In some embodiments, the organism, tissue, cell, or mixture endogenously contains miRNAs. In some embodiments, miRNAs are introduced into the organism, tissue, cell, or mixture. Such organisms, tissues, cells, or mixtures include those derived from animals, plants, and pathogens (e.g., viruses). Animals include, but are not limited to, vertebrates (e.g., amphibians (e.g., frog, newt, salamander, toad, etc.), birds (e.g., chicken, turkey, duck, pigeon, quail, swan, emu, ostrich, pheasant, etc.), fish (e.g., tuna, salmon, cod, tilapia, sardine, catfish, carp, pangasius, anchoveta, kingfish, pollock, etc.), mammals (e.g., human, dogs, cat, whale, dolphin, cattle, goat, pig, mouse, rat, ferret, llama, donkey, horse, rabbit, elephant, etc.), and reptiles (e.g., snake, gecko, chameleon, crocodile, alligator, turtle, etc.)) and invertebrates (e.g., ant, moth, fly, beetle, bee, termite, spider, shrimp, crab, octopus, worm, snail, etc.). Plants include, but are not limited to, algae, trees, mosses, fruit-producing plants, vegetable producing plants, oil-producing plants, nut-producing plants, corn, soybeans, barley, oats, sugar cane, rice, wheat, potatoes, tomatoes, grapes, cotton, applies, oranges, onions, cucumbers, bananas, melons, rapeseed, olives, sunflowers, spinach, lettuce, berries, tobacco, hemp, coffee, bamboo, peas, herbs, etc.

In some embodiments, a midR is generated from a miRNA from one organism priming RNA from a different organism. For example, in some embodiments, viral infected cells (e.g., human cells) may generate midRs and midPs using a miRNA from one organism to prime expression on the RNA of the other organism.

In some embodiments, identification and discovery is conducted, at least in part, in silico. Certain steps, operations, or processes described herein may be performed or implemented with one or more hardware or software modules, alone or in combination with other devices. In one embodiment, a software module is implemented with a computer program product comprising a computer-readable medium containing computer program code, which can be executed by a computer processor for performing any or all of the steps, operations, or processes described.

Embodiments of the invention may also relate to an apparatus for performing the operations herein. This apparatus may be specially constructed for the required purposes, and/or it may comprise a general-purpose computing device selectively activated or reconfigured by a computer program stored in the computer. Such a computer program may be stored in a non-transitory, tangible computer readable storage medium, or any type of media suitable for storing electronic instructions, which may be coupled to a computer system bus. Furthermore, any computing systems referred to in the specification may include a single processor or may be architectures employing multiple processor designs for increased computing capability.

Embodiments of the invention may also relate to a product that is produced by a computing process described herein. Such a product may comprise information resulting from a computing process, where the information is stored on a non-transitory, tangible computer readable storage medium and may include any embodiment of a computer program product or other data combination described herein.

In some embodiments, a method is provided wherein one or more miRNA sequences are matched, in silico, to one or more RNA sequences to form a predicted primer/template duplex. miRNA sequences may be obtained from databases (e.g., miRbase; currently having 38,589 entries; deepBase; microRNA.org; miRGen 4.0; PMRD (plant database); TargetScan; VIRmiRNA (viral database)), published literature, prediction algorithms, or identified experimentally. RNA sequences may be obtained from any number of databases, literature reference, or experimental sources (e.g., GenBank; RNAcentral; ARCHS4). Sequence alignment and structure analysis tools include, but are not limited to, BLAST, Infernal, R&R, PMFastR, and Stemloc.

One or more extension products from the miRNA primer is determined. In some embodiments, the one or more extension products are selected as having one or more IRES elements. In some embodiments, the one or more extension products are selected as having one or more start codons located in proximity to an IRES (e.g., within 10, 20, 50, 100, 200 nucleotides of the IRES). IRES base (See, Zhao et al. "IRESbase: A Comprehensive Database of Experimentally Validated Internal Ribosome Entry Sites," Genomics Proteomics Bioinformatics 18: 129-139 (2020)) provides a database of experimentally verified IRES sequences. In some embodiments, IRES prediction system are used (e.g., IRESpy, IRESPred, IRES finder, PatSearch).

In some embodiments, selected midR are reported (e.g., displayed on a computer monitor, printed, electronically communicated, etc.) to a user. In some embodiments, midP sequences are determined from one or more midR sequences. In some embodiments, identified midP sequences are reported to a user. In some embodiments, a machine learning system is employed to identify and select midR and/or midP sequences. In some embodiments, the machine learning system is trained using miRNA and RNA sequences that have been validated (e.g., validated to generate a midR found in one or more cell types, validated to encode for a midP that is found in one or more cell types, validated to encode a midR or midP associated with a disease cell, e.g., a cancer cell, an infected cell, etc.).

In some embodiments, a computer system reports the chemical structure of a midR or midP to a user. In some embodiments, the chemical structure comprises the sequence of the midR or midP. In some embodiments, the chemical structure identifies the terminal ends of a midR or midP (e.g., C-terminal end, N-terminal end). In some embodiments, the computer system comprises an electronic representation of one or more midR or midP sequences (e.g., any one or more or all of SEQ ID Nos: 1-19,712). The computer system may comprise a computer program for comparing the midR or midP sequences against databases of known sequences or against new sequencing queries (e.g., from a new candidate or identified midR or midP). In some embodiments, the computer system identifies, stores, and/or reports on matched pairs of midRs and midPs, optionally with their associated mRNAs and miRNAs.

In some embodiments, identification and discovery is conducted, at least in part, employing one or more biological or chemical laboratory experiments. Laboratory experiments include, but are not limited to, amplification of nucleic acid from a sample (e.g., one more cells) using nucleic acid primers (e.g., miRNAs or sequences derived from miRNAs). In some embodiments, the primers are identified by, or selected using, an in-silico method. In some embodiments, laboratory experiments comprise nucleic acid capture employing one or more probes attached to a solid surface. In some embodiments, the probe is a nucleic acid sequence complementary to a midR. In some embodiments, the probe sequence is selected to capture, via hybridization, a midR, but not a corresponding miRNA. In some embodiments, the laboratory experiments comprise capture of one or more midPs employing binding agents that selectively bind midPs. For example, in some embodiments, an antibody or other binding agent that is selected for one or more midPs is attached to a solid surface and a sample suspected of having a midP is contacted to the surface, followed by washing or other steps to remove non-specifically bound molecules.

### midPs

In some embodiments, provided herein are midPs and compositions, methods, systems, and kits comprising or employing one or more midPs.

In some embodiments, isolated midPs are provided. The isolated midPs maybe be purified from natural sources. In some embodiments, the isolated midP is synthetized enzymatically (e.g., recombinantly) or chemically in a laboratory. In some embodiments, the midP is a variant midP. In some such embodiments, the midP contains one or more amino acid substitutions, deletions, or additions compared to a wild-type midP.

The present invention contemplates several types of compositions which involve polypeptide variants and derivatives. These include substitutional, insertional, deletion and covalent variants and derivatives. The term "derivative" is used synonymously with the term "variant" but generally refers to a molecule that has been modified and/or changed in any way relative to a reference molecule or starting molecule.

As such, polynucleotides encoding peptides or polypeptides containing substitutions, insertions and/or additions, deletions and covalent modifications with respect to reference sequences, in particular the polypeptide sequences disclosed herein, are included within the scope of this invention. For example, sequence tags or amino acids, such as one or more lysines, can be added to the peptide sequences of the invention (e.g., at the N-terminal or C-terminal ends). Sequence tags can be used for peptide purification or localization. Lysines can be used to increase peptide solubility or to allow for biotinylation. Alternatively, amino acid residues located at the carboxy- and amino-terminal regions of the amino acid sequence of a peptide or protein may optionally be deleted providing for truncated sequences. Certain amino acids (e.g., C-terminal or N-terminal residues) may alternatively be deleted depending on the use of the sequence, as for example, expression of the sequence as part of a larger sequence which is soluble, or linked to a solid support.

"Substitutional variants" when referring to polypeptides are those that have at least one amino acid residue in a native or starting sequence removed and a different amino acid inserted in its place at the same position. The substitutions may be single, where only one amino acid in the molecule has been substituted, or they may be multiple, where two or more amino acids have been substituted in the same molecule.

As used herein the term "conservative amino acid substitution" refers to the substitution of an amino acid that is normally present in the sequence with a different amino acid of similar size, charge, or polarity. Examples of conservative substitutions include the substitution of a non-polar (hydrophobic) residue such as isoleucine, valine and leucine for another non-polar residue. Likewise, examples of conservative substitutions include the substitution of one polar (hydrophilic) residue for another such as between arginine and lysine, between glutamine and asparagine, and between glycine and serine. Additionally, the substitution of a basic residue such as lysine, arginine or histidine for another, or the substitution of one acidic residue such as aspartic acid or glutamic acid for another acidic residue are additional examples of conservative substitutions. Examples of non-conservative substitutions include the substitution of a non-polar (hydrophobic) amino acid residue such as isoleucine, valine, leucine, alanine, methionine for a polar (hydrophilic) residue such as cysteine, glutamine, glutamic acid or lysine and/or a polar residue for a non-polar residue.

"Insertional variants" when referring to polypeptides are those with one or more amino acids inserted immediately adjacent to an amino acid at a particular position in a native or starting sequence. "Immediately adjacent" to an amino acid means connected to either the alpha-carboxy or alpha-amino functional group of the amino acid.

"Deletional variants" when referring to polypeptides are those with one or more amino acids in the native or starting amino acid sequence removed. Ordinarily, deletional variants will have one or more amino acids deleted in a particular region of the molecule.

"Covalent derivatives" when referring to polypeptides include modifications of a native or starting protein with an organic proteinaceous or non-proteinaceous derivatizing agent, and/or post-translational modifications. Covalent modifications are traditionally introduced by reacting targeted amino acid residues of the protein with an organic derivatizing agent that is capable of reacting with selected side-chains or terminal residues, or by harnessing mechanisms of post-translational modifications that function in selected recombinant host cells. The resultant covalent derivatives are useful in programs directed at identifying residues important for biological activity, for immunoassays, or for the preparation of anti-protein antibodies for immunoaffinity purification of the recombinant glycoprotein.

Certain post-translational modifications are the result of the action of recombinant host cells on the expressed polypeptide. Glutaminyl and asparaginyl residues are frequently post-translationally deamidated to the corresponding glutamyl and aspartyl residues. Alternatively, these residues are deamidated under mildly acidic conditions. Either form of these residues may be present in the polypeptides produced in accordance with the present invention.

Other post-translational modifications include hydroxylation of proline and lysine, phosphorylation of hydroxyl groups of seryl or threonyl residues, methylation of the alpha-amino groups of lysine, arginine, and histidine side chains.

As recognized by those skilled in the art, protein fragments, functional protein domains, and homologous proteins are also considered to be within the scope of polypeptides of interest of this invention. For example, provided herein is any protein fragment (meaning a polypeptide sequence at least one amino acid residue shorter than a reference polypeptide sequence but otherwise identical) of a reference protein 10, 20, 30, 40, 50, 60, 70, 80, 90, 100 or greater than 100 amino acids in length. In another example, any protein that includes a stretch of about 10, 20, about 30, about 40, about 50, or about 100 amino acids which are about 40%, about 50%, about 60%, about 70%, about 80%, about 90%, about 95%, or about 100% identical to any of the sequences described herein can be utilized in accordance with the invention. In certain embodiments, a polypeptide to be utilized in accordance with the invention includes 2, 3, 4, 5, 6, 7, 8, 9, 10, or more mutations as shown in any of the sequences provided or referenced herein.

In some embodiments, midPs may be produced (e.g., recombinantly) as a fusion protein. In some embodiments, the midP fusion partner is another midP. In some embodiments, the midP fusion partner is purification tag (e.g., polyhistidine (e.g., hexahistidine), GST, Myc, MBP, CBP, biotin) or detectable label (e.g., fluorescent fusion protein (e.g., GFP)). In some embodiments, the fusion partner is an antibody or antibody fragment. In some embodiments, the fusion partner is one sub-component of a multi-component complementation protein. In some embodiments, the fusion partner is a targeting protein that directs the midP to a particular location (e.g., cancer cell targeting, organelle targeting, cell membrane targeting, etc.). In some embodiments, the midP is indirectly connected to the fusion partner through a linker. In some embodiments, the fusion partner is a lipid, carbohydrate, nucleic acid, small molecule (e.g., drug, label, etc.), or other non-peptide molecule.

In some embodiments the midP is expressed in or introduced into a cell, such that the midP is a heterologous polypeptide in the cell. For example, a midP from a first organism (e.g., mammalian, plant, etc.) may be expressed in or introduced into a cell from a different organism. Host cells may be used as production systems for making large amounts of a midP. Host cells may also be used to test the biological activity of midPs. Suitable host cells include, but are not limited to, *E. coli, Lactococcus lactis, Saccharomyces cerevisiae, Schizosaccharomyces pombe, Pichia pastoris,* HEK 293, COS, S2, CHO, NSO, DT40 and the like.

In some embodiments, one or more midPs are attached to a solid support. For example, in some embodiments, one or more (e.g., 1, 2, 5, 10, 20, 50, 100, 200, 500, 1000, 2000 or more) midPs are attached to one or more solid supports to provide a protein array. Protein arrays find use, for example, for the detection and analysis of molecules that bind to midPs. Such molecules may include, but are not limited to, detection of natural or heterologous binding partners, detection of antibodies, detection of autoantibodies, and the like. In some embodiments, one or more midPs are attached directly or indirectly (e.g., via a linker) to a surface plasmon resonance surface. The midPs are contacted with a sample suspected of containing a midP-binding molecule and analyzed by surface plasmon resonance.

In some embodiments, a reaction mixture or solid support comprises one or more midP-specific binding partners (e.g., antibodies, etc.) for use in detecting the presence of one or more midPs in a sample. In some embodiments, systems and reaction mixtures comprise midPs bound to a binding partner. Suitable formats include, but are not limited to, microarrays, microwell plates, microfluidic cartridges, lateral flow devices, capture beads, slides, flow cells, and the like.

In some embodiments, one or more midPs comprising a label are provided. Labeled midPs find use, for example, to identify midP binding partners (e.g., peptides, proteins, nucleic acids, or other molecules that bind to midPs). For example, labeled midPs may be contacted with a sample and analyzed using an electrophoretic mobility shift assay (EMSA) to identify factors that localize with midP. In some embodiments, two or more fragments of a midP are used in a complementation assay. In some embodiments, one or more midPs are contacted with free radicals (e.g., hydroxyl radicals), optionally exposed to a sample of interest, and then analyzed (e.g., via digestion and LC/MS).

In some embodiments, midPs or antigenic portions thereof, are employed in immunogenic compositions or methods. In some such embodiments, the compositions and methods employ an adjuvant and/or immunostimulant. Adjuvants and immunostimulants are compounds that either directly or indirectly stimulate the immune system's response to a co-administered antigen. Suitable adjuvants are commercially available as, for example, Freund's Incomplete Adjuvant and Complete Adjuvant (Difco Laboratories, Detroit, Mich.); Merck Adjuvant 65 (Merck and Company, Inc., Rahway, N.J.); AS-2 (SmithKline Beecham); mineral salts (for example, aluminum, silica, kaolin, and carbon); aluminum salts such as aluminum hydroxide gel (alum), AlK(SO4)2, AlNa(SO4)2, AlNH4(SO4), and Al(OH)3; salts of calcium (e.g., Ca3(PO4)2), iron or zinc; an insoluble suspension of acylated tyrosine; acylated sugars; cationically or anionically derivatized polysaccharides; polynucleotides (for example, poly IC and poly AU acids); polyphosphazenes; cyanoacrylates; polymerase-(DL-lactide-co- glycoside); biodegradable microspheres; liposomes; lipid A and its derivatives; monophosphoryl lipid A; wax D from Mycobacterium tuberculosis, as well as substances found in Corynebacterium parvum, Bordetella pertussis, and members of the genus Brucella); bovine serum albumin; diphtheria toxoid; tetanus toxoid; edestin; keyhole-limpet hemocyanin; Pseudomonal Toxin A; choleragenoid; cholera toxin; pertussis toxin; viral proteins; and Quil A. In addition, adjuvants such as cytokines (e.g., GM-CSF or interleukin-2, -7, or -12), interferons, or tumor necrosis factor, may also be used as adjuvants.

In some embodiments, a kit is provided comprising one or more midPs. The kit may further comprise one or more reagents, materials, or other components necessary, sufficient, or useful to practice any of the methods described herein or to generate any of the compositions or systems described herein. For example, the kits may comprise one or more or all of: a label, a solid support, a detection instrument, a host cell, positive and negative control samples, software, instructions, and the like.

### midRs

In some embodiments, provided herein are midRs and compositions, methods, systems, and kits comprising or employing one or more midRs.

In some embodiments, isolated midRs are provided. The isolated midPs maybe be purified from natural sources. In some embodiments, the isolated midR is synthetized enzymatically or chemically in a laboratory. In some embodiments, the midR is a variant midR. In some such embodiments, the midR contains one or more base substitutions, deletions, or additions compared to a wild-type midR.

In some embodiments, DNA sequences corresponding to or complementary to the midR sequences are provided. In some embodiments, the DNA sequence comprises, consists essentially of, or consists of the DNA version of the midR sequence (having thymine (T) in place of uracil). In some embodiments, the DNA sequence shares the same 5' and 3' terminal bases with its corresponding midR. In some embodiments, the DNA sequence comprises, consists essentially of, or consists of the DNA complement of the midR sequence. In some embodiments, the DNA sequence corresponds to or is complementary to a sub-region of a midR (e.g., a midR fragment). DNA sequences may be generated that encode a midR. In some embodiments, the DNA sequences are used as primers to amplify midR sequences or as probes to detect midR sequences. In some embodiments, primers and/or probes are designed to identify the 5' and or 3' terminal ends of a midR to differentiate the midR from other sequences that to not share such termini.

In some embodiments variant midR or variant DNA corresponding to a midR or complementary to a midR is provided. In some embodiments, the variant is at least 70%, 75%, 80%, 85%, 90%, 91%, 92%, 93%, 94%, 95%, 96%, 97%, 98% or 99% identical to any of SEQ ID Nos: 8260-19,712, or complement thereof, or a fragment thereof having at least 20, 50, 100, 200, or 500 bases thereof. In some embodiments, the variant is codon-optimized for optimal expression of the midR in a particular host cell of interest.

In some embodiments, a vector comprising a midR, or DNA corresponding to or complementary to a midR, is provided. In some embodiments, the vector is an expression vector that encodes a midR. In some embodiments, the vector is a gene transfer vector that integrates a midR or sequencing expressing a midR into a cell (e.g., integrated into a genome or expressed episomally). The present invention is not limited to particular expression vectors. Exemplary vectors and expression methods are described herein.

In some embodiments, midRs and/or midPs are expressed using any suitable vector and expression system. In some embodiments, midRs and/or midPs are expressed in bacterial or eukaryotic expression vectors (e.g., commercially available vectors). In some embodiments, midRs and/or midPs are expressed in viral (e.g., adeno, adeno-associated, retro- or lenti-viral) vectors. Suitable vectors are introduced into suitable host cells (e.g., bacterial or eukaryotic host cells), expression is induced, and RNA or peptides are isolated using any suitable method.

The midRs and/or midPs of the present invention may be produced by recombinant techniques. Thus, for example, a polynucleotide encoding a peptide, polypeptide or protein of the present invention may be included in any one of a variety of expression vectors for expressing a midRs and/or midPs. In some embodiments of the present invention, vectors include, but are not limited to, chromosomal, nonchromosomal and synthetic DNA sequences (e.g., derivatives of SV40, bacterial plasmids, phage DNA; baculovirus, yeast plasmids, vectors derived from combinations of plasmids and phage DNA, retroviral vectors and viral DNA such as vaccinia, adenovirus, fowl pox virus, and pseudorabies). It is contemplated that any vector may be used as long as it is viable in the host.

In particular, some embodiments of the present invention provide recombinant constructs comprising one or more of the sequences as broadly described above. In some embodiments of the present invention, the constructs comprise a vector, such as a plasmid or viral vector, into which a sequence of the invention has been inserted, in a forward or reverse orientation. In still other embodiments, the heterologous structural sequence is assembled in appropriate phase with translation initiation and termination sequences. In preferred embodiments of the present invention, the appropriate sequence is inserted into the vector using any of a variety of procedures. In general, the sequence is inserted into an appropriate restriction endonuclease site(s) by procedures known in the art.

Large numbers of suitable vectors are known to those of skill in the art, and are commercially available. Such vectors include, but are not limited to, the following vectors: 1) Bacterial-pQE70, pQE60, pQE-9 (Qiagen), pBS, pD10, phagescript, psiX174, pbluescript SK, pBSKS, pNH8A, pNH16a, pNH18A, pNH46A (Stratagene); ptrc99a, pKK223-3, pKK233-3, pDR540, pRIT5 (Pharmacia); 2) Eukaryotic-pWLNEO, pSV2CAT, pOG44, PXT1, pSG (Stratagene) pSVK3, pBPV, pMSG, pSVL (Pharmacia); and 3) Baculovirus-pPbac and pMbac (Stratagene). Any other plasmid or vector may be used as long as they are viable in the host. In some preferred embodiments of the present invention, mammalian expression vectors comprise an origin of replication, a suitable promoter and enhancer, and also any necessary ribosome binding sites, polyadenylation sites, splice donor and acceptor sites, transcriptional termination sequences, and 5' flanking non-transcribed sequences.

In certain embodiments of the present invention, the DNA sequence in the expression vector is operatively linked to an appropriate expression control sequence(s) (promoter) to direct RNA synthesis. Promoters useful in the present invention include, but are not limited to, the LTR or SV40 promoter, the *E. coli* lac or trp, the phage lambda P_{L} and P_{R}, T3 and T7 promoters, and the cytomegalovirus (CMV) immediate early, herpes simplex virus (HSV) thymidine kinase, and mouse metallothionein-I promoters and other promoters known to control expression of gene in prokaryotic or eukaryotic cells or their viruses. In some embodiments, inducible promoters are employed to allow temporal control over the timing of expression.

In some embodiments, nucleic acid molecules are provided that alter the expression of or function of midRs. For example, in some embodiments, capture vectors are provided the bind to miRNAs and prevent generation of midRs or midPs in a cell. In some embodiments, antisense oligonucleotide or RNAi molecules are provided that inhibit the expression of midRs or midPs in a reaction mixture, cell, tissue, or organism.

In some embodiments, non-natural modified nucleotides may be introduced to polynucleotides or nucleic acids during synthesis or post-synthesis of the chains to achieve desired functions or properties. The modifications may be on internucleotide lineage, the purine or pyrimidine bases, or sugar. The modification may be introduced at the terminal of a chain or anywhere else in the chain; with chemical synthesis or with a polymerase enzyme. For example, hexitol nucleic acids (HNAs) are nuclease resistant and provide strong hybridization to RNA. In some embodiments, a chimeric HNA gapmer oligonucleotide comprising a phosphorothioate central sequence flanked by 5' and 3' HNA sequences is employed (See e.g., Kang et al., Nucleic Acids Research, vol. 32(4), 4411-4419 (2004), the contents of which are incorporated herein by reference in their entirety). The preparation and uses of modified nucleotides comprising 6-member rings in RNA interference, antisense therapy or other applications are disclosed in US Pat. Application No. 2008/0261905, US Pat. Application No. 2010/0009865, and International Publication No. WO97/30064 to Herdewijn et al.; the contents of each of which are herein incorporated by reference in their entireties).

In some embodiments, midRs may be produced (e.g., recombinantly) as a fusion nucleic acid (e.g., encoding a fusion protein). In some embodiments, the midR fusion partner is another midR. In some embodiments, the midR fusion partner encodes a purification tag (e.g., polyhistidine (e.g., hexahistidine), GST, Myc, MBP, CBP, biotin) or detectable label (e.g., fluorescent fusion protein (e.g., GFP)).

In some embodiments the midR is expressed in or introduced into a cell, such that the midR is a heterologous polynucleotide in the cell. For example, a midR from a first organism (e.g., mammalian, plant, etc.) may be expressed in or introduced into a cell from a different organism.

In some embodiments, one or more midRs, fragments thereof, or sequences complementary thereto, are attached to a solid support. For example, in some embodiments, one or more (e.g., 1, 2, 5, 10, 20, 50, 100, 200, 500, 1000, 2000 or more) such sequences are attached to one or more solid supports to provide a nucleic acid array. Nucleic acid arrays find use, for example, for the detection and analysis of midRs or molecules that bind to midRs.

In some embodiments, a reaction mixture or solid support comprises one or more midR specific binding partners (e.g., capture oligonucleotides, etc.) for use in detecting the presence of one or more midRs in a sample. In some embodiments, systems and reaction mixtures comprise midRs bound to a binding partner. Suitable formats include, but are not limited to, microarrays, microwell plates, microfluidic cartridges, lateral flow devices, capture beads, slides, flow cells, and the like. In some embodiments, midRs, fragments thereof, midR capture probes are attached to a solid support directly or through a linker.

In some embodiments, one or more midRs, fragments thereof, or sequences complementary thereto comprise a label. Labeled molecules find use, for example, to identify midRs and midR binding partners. In some embodiments, labeled molecules find use as primers or probes.

Either enzymatic or chemical ligation methods can be used to conjugate polynucleotides or their regions with different functional blocks, such as labels, solid supports, nanoparticles, delivery agents, etc. In some embodiments, amplification employs one or more primers, a replication enzyme (e.g., a polymerase), dideoxy nucleotides, and appropriate buffers and instrumentation. In some embodiments, cDNA is generated from RNA prior to amplification, for example, by use of a reverse transcriptase enzyme.

In some embodiments, midRs, fragments thereof, or cDNA copies thereof may be amplified. Amplification may be exponential or linear and may involved temperature cycling or may be isothermal. In some embodiments, amplification comprises polymerase chain reaction (PCR) (e.g., digital PCR and droplet digital PCR (ddPCR), quantitative PCR, real time PCR, multiplex PCR, PCR-based singleplex methods, emulsion PCR), and/or isothermal amplification.

In some embodiments, systems, compositions, and methods for sequencing midRs are provided. In some embodiments, a barcode is added to a midR, a fragment thereof, or a cDNA copy thereof. In some embodiments, a barcode sequence is ligated to the midR, a fragment thereof, or a cDNA copy thereof. In some embodiments, a midR is hybridized to a capture comprising a barcode sequence. Barcodes can have a variety of different formats. For example, barcodes can include polynucleotide barcodes, random nucleic acid sequences, and synthetic nucleic acid sequences. A barcode can be attached to an analyte or to another moiety or structure in a reversible or irreversible manner. A barcode can be added to, for example, a fragment of a deoxyribonucleic acid (DNA) or ribonucleic acid (RNA) sample before or during sequencing of the sample. Barcodes can allow for identification and/or quantification of individual sequencing-reads (e.g., a barcode can be or can include a unique molecular identifier or "UMI"). Barcodes can spatially-resolve molecular components found in biological samples, for example, at single-cell resolution (e.g., a barcode can be or can include a "spatial barcode"). In some embodiments, a barcode includes both a UMI and a spatial barcode.

In some embodiments, one or more adapters is added to a midR, a fragment thereof, or a cDNA copy thereof. Adaptors can be added using any one of many different techniques including, but not limited to, ligation, hybridization, and tagmentation. Adaptors can also be nucleic acid sequences that add a function, e.g., spacer sequences, primer sequences/sites, barcode sequences, unique molecular identifier sequences. In some embodiments, the adaptor is designed for use with a specific sequencing platform: e.g., includes at least a portion of a nucleic acid domain (e.g., a sequencing platform adapter nucleic acid sequence) or complement thereof utilized by a sequencing platform of interest, such as a sequencing platform provided by Illumina^{®} (e.g., the NovaSeq^{™}, NexSeq^{™}, HiSeq^{™}, MiSeq^{™} and/or Genome Analyzer^{™} sequencing systems); Thermo Fisher (e.g., Ion Torrent^{™} (such as the Ion PGM^{™} and/or Ion Proton^{™} sequencing systems) and Life Technologies^{™} (such as a SOLiD sequencing system)); Pacific Biosciences (e.g., the PACBIO RS II sequencing system); Roche (e.g., the 454 GS FLX+ and/or GS Junior sequencing systems); Oxford Nanopore technologies (e.g., MinION^{™}, GridION^{™}' PrometION^{™} sequencing systems) or any other sequencing platform of interest. In some embodiments, the sequencing platform adapter construct includes a nucleic acid domain selected from: a domain (e.g., a capture site or capture sequence) that specifically binds to a surface-attached sequencing platform oligonucleotide (e.g., the P5/i5 or P7/i7 oligonucleotides attached to the surface of a flow cell in an Illumina^{®} sequencing system).

Sequencing of polynucleotides can be performed by various commercial systems. Examples of methods for sequencing genetic material include, but are not limited to, DNA hybridization methods (e.g., Southern blotting), restriction enzyme digestion methods, Sanger sequencing methods, next-generation sequencing methods (e.g., single-molecule real-time sequencing, nanopore sequencing, and Polony sequencing), ligation methods, and microarray methods. Additional examples of sequencing methods that can be used include targeted sequencing, single molecule real-time sequencing, electron microscopy-based sequencing, panel sequencing, transistor-mediated sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, co-amplification at lower denaturation temperature-PCR (COLD-PCR), sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD^{™} sequencing (sequencing by oligonucleotide ligation and detection), MS-PET sequencing, and any combinations thereof.

Sequence analysis of the nucleic acid molecules (including barcoded nucleic acid molecules or derivatives thereof) can be direct or indirect. Thus, the sequence analysis substrate (which can be viewed as the molecule which is subjected to the sequence analysis step or process) can directly be the barcoded nucleic acid molecule or it can be a molecule which is derived therefrom (e.g., a complement thereof). Thus, for example, in the sequence analysis step of a sequencing reaction, the sequencing template can be the barcoded nucleic acid molecule or it can be a molecule derived therefrom.

In some embodiments, direct sequencing of captured RNA is performed by sequencing-by-synthesis (SBS). In some embodiments, a sequencing primer is complementary to a sequence in one or more of the domains of a capture probe (e.g., functional domain). In such embodiments, sequencing-by-synthesis can include reverse transcription and/or amplification in order to generate a template sequence (e.g., functional domain) from which a primer sequence can bind.

SBS techniques which can be used are described for example, but not limited to, those in U.S. Patent App. Pub. No. 2007/0166705, U.S. Patent App. Pub. No. 2006/0188901, U.S. Pat. No. 7,057,026, U.S. Patent App. Pub. No. 2006/0240439, U.S. Patent App. Pub. No. 2006/0281109, PCT Patent App. Pub. No. WO 05/065814, U.S. Patent App. Pub. No. 2005/0100900, PCT Patent App. Pub. No. WO 06/064199, PCT Patent App. Pub. No. WO07/010,251, U.S. Patent App. Pub. No. 2012/0270305, U.S. Patent App. Pub. No. 2013/0260372, and U.S. Patent App. Pub. No. 2013/0079232, the entire contents of each of which are incorporated herein by reference.

In some embodiments, an RNA is directly sequenced using a nanopore-based method. In some embodiments, direct sequencing is performed using nanopore direct RNA sequencing in which captured RNA is translocated through a nanopore. A nanopore current can be recorded and converted into a base sequence. In some embodiments, captured RNA remains attached to a substrate during nanopore sequencing. In some embodiments, captured RNA is released from the substrate prior to nanopore sequencing. Examples of nanopore-based sequencing methods that can be used are described in Deamer et al., Trends Biotechnol. 18, 14 7-151 (2000); Deamer et al., Acc. Chem. Res. 35:817-825 (2002); Li et al., Nat. Mater. 2:611-615 (2003); Soni et al., Clin. Chem. 53, 1996-2001 (2007); Healy et al., Nanomed. 2, 459-481 (2007); Cockroft et al., J. Am. Chem. Soc. 130, 818-820 (2008); and in U.S. Pat. No. 7,001,792. The entire contents of each of the foregoing references are incorporated herein by reference.

In some embodiments, a kit is provided comprising one or more midRs, fragments thereof, DNA sequences corresponding to or complementary to a midR, and vectors comprising such sequences. The kit may further comprise one or more reagents, materials, or other components necessary, sufficient, or useful to practice any of the methods described herein or to generate any of the compositions or system described herein. For example, the kits may comprise one or more or all of: a label, a solid support, a detection instrument, a host cell, analysis reagents, positive and negative control samples, software, instructions, and the like.

### Uses

The systems, compositions, methods, and kits provided herein find use in research, industrial, and clinical methods.

Research methods include, but are not limited to, identification of midRs and/or midPs in silico and in biological and environmental samples; detection of midRs and/or midPs in samples; chemical synthesis of midRs and/or midPs; generation of solid supports comprising midRs and/or midPs or molecules designed to bind thereto (e.g., for use as arrays for detecting midRs and/or midPs and/or molecules that bind thereto, such as antibodies, proteins, etc.); and biomarkers for unique fingerprinting or other characterization of cells, tissues, or organisms (e.g., which can be used in combination with other classes of biological information to provide the most definitive identification of biological materials (e.g., cells, exosomes, fluids, tissues, etc.) or their biological status-including, but not limited to, DNA sequences, mutations analysis, expression profiles, epigenetic information, proteomic information, metabolomic information, etc.).

In some embodiments, midRs and/or midPs are used for screening or diagnostic purposes. For example, as disclosed herein, many midRs and midPs are associated with disease states (e.g., cancer). Detection of midRs and/or midPs provides information that finds use on its own or use in combination with other information (e.g., genomic, proteomic, epigenetic, metabolic, etc.) to identify or predict a metabolic or disease state. midRs and midPs may be detected in any sample type. Nucleic acids and polypeptides may be detected by any suitable technique including, but not limited to, amplification, sequencing, microarray analysis, mass spectroscopy, ELISA, microscopy, affinity binding, crystallography, and the like.

In some embodiments, midRs and/or midPs, or agents altering their expression or function, are used therapeutically, either as drug discovery agents or as therapeutic agents themselves. In some embodiments, a midR or midP is administered to a cell, tissue, or subject. In some embodiments, an agent that inhibits one or more midRs or midPs is administered to a cell, tissue, or subject. For example, in some embodiments, a molecule that binds to and sequesters miRNAs is administered to a cell, tissue, or subject. In some embodiments, an antisense or RNAi molecule that binds to and cleaves RNA, midR, or blocks RNA/miRNA interaction is administered to a cell, tissue, or subject. In some embodiments, an aptamer, antibody, or other selective binder that binds to and inhibits a midR and/or midP is administered to a cell, tissue, or subject. In some embodiments, the agent is an agonist that increases the expression and/or activity of a midR and/or midP.

In some embodiments, the subject has cancer or is suspected of having cancer, such as prostate cancer, melanoma, bladder cancer, breast cancer, lymphoma, ovarian cancer, lung cancer, colorectal cancer or head and neck cancer. In some embodiments, the subject has an immunological disease or disorder or is suspected of having an immunological disease or disorder. In some embodiments, the subject has an inflammatory disease or disorder or is suspected of having an inflammatory disease or disorder. In some embodiments, the subject has a neurogenerative disease or disorder or is suspected of having a neurogenerative disease or disorder. In some embodiments, the subject has a metabolic disease or disorder or is suspected of having metabolic disease or disorder.

As used herein, the terms "treat," treating," "treatment," and the like, are meant to decrease, suppress, attenuate, diminish, arrest, the underlying cause of a disease, disorder, or condition, or to stabilize the development or progression of a disease, disorder, condition, and/or symptoms associated therewith. The terms "treat," "treating," "treatment," and the like, as used herein can refer to curative therapy, prophylactic therapy, and preventative therapy. The treatment, administration, or therapy can be consecutive or intermittent. Consecutive treatment, administration, or therapy refers to treatment on at least a daily basis without interruption in treatment by one or more days. Intermittent treatment or administration, or treatment or administration in an intermittent fashion, refers to treatment that is not consecutive, but rather cyclic in nature. Treatment according to the presently disclosed methods can result in complete relief or cure from a disease, disorder, or condition, or partial amelioration of one or more symptoms of the disease, disease, or condition, and can be temporary or permanent. The term "treatment" also is intended to encompass prophylaxis, therapy and cure.

As used herein, the terms "prevent," "preventing," "prevention," "prophylactic treatment" and the like refer to reducing the probability of developing a disease, disorder, or condition in a subject, who does not have, but is at risk of or susceptible to developing a disease, disorder, or condition. Thus, in some embodiments, an agent can be administered prophylactically to prevent the onset of a disease, disorder, or condition, or to prevent the recurrence of a disease, disorder, or condition.

The term "effective amount," as in "a therapeutically effective amount," of a therapeutic agent refers to the amount of the agent necessary to elicit the desired biological response. As will be appreciated by those of ordinary skill in this art, the effective amount of an agent may vary depending on such factors as the desired biological endpoint, the agent to be delivered, the composition of the pharmaceutical composition, the target tissue or cell, and the like. More particularly, the term "effective amount" refers to an amount sufficient to produce the desired effect, e.g., to reduce or ameliorate the severity, duration, progression, or onset of a disease, disorder, or condition, or one or more symptoms thereof; prevent the advancement of a disease, disorder, or condition, cause the regression of a disease, disorder, or condition; prevent the recurrence, development, onset or progression of a symptom associated with a disease, disorder, or condition, or enhance or improve the prophylactic or therapeutic effect(s) of another therapy.

As will be appreciated by one of ordinary skill in the art, many factors influence the choice of appropriate dosage and schedule of administration. For example, the appropriate dosage for achieving a desired therapeutic effect in a subject may depend on whether the subject is a human, or another mammal, or is a non-mammalian subject; it may depend on the subject's age, weight, sex, diet, health, underlying medical condition, and the like. Therefore, a physician, veterinarian, or other medical, science, or health practitioner skilled in the art will be able to devise, in light of the guidance provided herein, and without undue experimentation, an appropriate treatment protocol for practicing the presently disclosed methods.

Actual dosage levels of the active ingredients in the presently disclosed pharmaceutical compositions can be varied so as to obtain an amount of the active ingredient that is effective to achieve the desired therapeutic response for a particular subject, composition, route of administration, and disease, disorder, or condition without being toxic to the subject. The selected dosage level will depend on a variety of factors including the activity of the particular compound employed, or salt thereof, the route of administration, the time of administration, the rate of excretion of the particular compound being employed, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the agents for use within the methods of the presently disclosed subject matter at levels lower than that required to achieve the desired therapeutic effect and gradually increase the dosage until the desired effect is achieved. Accordingly, the dosage range for administration will be adjusted by the physician as necessary. It will be appreciated that an amount of a compound required for achieving the desired biological may be different from the amount of compound effective for another purpose.

### EXAMPLES

### Methods

### Bioinformatics-based prediction of midRs and midPs

2,656 human miRNAs were obtained from the miRbase website. The 3'-end (e.g., 15-nt fragment) of each miRNA (U→T) was searched against the NCBI RefSeq_RNA database (accessed on December 20, 2020) using blastn (version 2.11.0+), to identify paired RNA templates to generate midRs:
blastn -task blastn-short -db db_refseq_rna -query query_15nt.fa -out out_15nt.xml - outfmt 5 -evalue 1 -gapopen 5 -gapextend 2 -reward 1 -penalty -3 -ungapped - max_target_seqs 10000 -strand minus -taxids 9606

Since the queries are short, the option "-task blastn-short" was used. Default parameters "-gapopen 5 -gapextend 2 -reward 1 -penalty -3" were applied for "blastn-short" tasks. The option "-ungapped" ensures no gap in alignments. The option "-strand minus" restricts the search to reverse matches. The option "-taxids 9606" restricts the search to human RNAs. For 15-nt queries, the calculated e-value for an exact, ungapped 15-nt match is 0.941241 and hence a slightly higher e-value cutoff "-evalue 1" was used. Similarly, the e-value cutoffs were set to 5, 20, 100, 500, and 1,000 for 14-, 13-, 12-, 11-, and 10-nt queries, respectively. With these parameters, a sufficiently high cutoff ("-max_target_seqs 10000") was set to output RNA hits; this threshold was not reached for the 10- to 15-nt queries. For X-nt queries, the searched RNA hits were further filtered to retain those with exact X-nt match. For the sake of description, below the 15-nt match is used as an example. The information for a hit such as the GenInfo Identifier (GI) number, NCBI Reference Sequence number, start and end matching positions, and gene symbol were recorded.

The RNA sequence of a hit was retrieved using its GI:
blastdbcmd -entry gi -db db_refseq_rna

The midR sequence was generated by reverse transcribing the hit RNA from the end matching position until its 5'-end. The number of midRs derived by each human miRNA varies substantially. For instance, some miRNAs (e.g., hsa-miR-99b-5p, hsa-miR-9985, hsa-miR-9903, etc.) do not yield any midR while has-miR-619-5p alone derives 3,422 midRs. A total of 13,310 midRs were derived from all 2,656 human miRNAs (Table 1). Among them, 11,453 unique midRs were used to create a database:
mkblastdb -in db_RNA_uniq.fa -out db_RNA_uniq -dbtype nucl

The unique midRs were translated into proteins following two criteria: (1) a midR must have a predicted IRES (11) and (2) a start codon is available within 100 nt downstream the IRES. The first criterion was checked by comparing midRs with the experimental IRES sequences taken from IRESbase, which contains 554 viral IRESs, 691 human IRESs, and 83 IRESs from other eukaryotes. Because the length of IRESs varies substantially, they were searched against the midR database with different blastn parameters.
if an IRES has ≤15 nucleotides:
   blastn -db db_ uniq_midr -query ires_le15.fa -out ires_le15.xml -outfmt 5 -evalue 1e5 - task blastn-short -max_target_seqs 2500000 -reward 1-penalty -3 -gapopen 5 -gapextend 2 -ungapped -strand plus
else if an IRES has <50 nucleotides:
   blastn -db db_ uniq_midr -query ires_lt50.fa -out ires_lt50.xml -outfmt 5 -evalue 1e5 - task blastn-short -max_target_seqs 2500000 -reward 1 -penalty -2 -gapopen 0 -gapextend 2 -strand plus
otherwise:
   blastn -db db_ uniq_midr -query ires_ge50.fa -out ires_ge50.xml -outfmt 5 -evalue 1e5 - task blastn -max_target_seqs 2500000 -reward 2 -penalty -3 -gapopen 5 -gapextend 2 - strand plus

The option "-strand plus" restricts the search to forward matches. For very short IRES queries (≤15 nt), high gap opening penalty "-gapopen 5" and "-ungapped" were used to disallow gaps; "-penalty -3" was used to disfavor mismatches. For IRES queries in (15, 50) nt, the penalty scores were reduced to allow more mismatches and gap openings. For >50-nt IRES queries, default parameters for a conventional "blastn" task were used. In all cases, high cutoffs for e-value ("-evalue 1e5") and number of targets ("-max_target_seqs 2500000", larger than the number of midRs) were used.

An IRES site was predicted to be existing if coverage ≥1 and aligned identity ≥1 for ≤15-nt IRES queries, or coverage ≥0.7 and aligned identity ≥0.75 for >15-nt IRES queries. Here, the coverage and aligned identity were defined as coverage=(aligned length)/(query length) and aligned identity=identity/(aligned length), and the aligned length, query length, and identity were extracted from the blastn output.

The midRs were then translated following the second criterion. A midR may contain multiple IRESs and following an IRES may exist multiple start codons. All possibilities were considered when translating a midR into midP(s). A total of 5,369 midPs were obtained with >10 amino acids, of which 1,239 are unique. The midP sequences were searched against the NCBI nr protein database (accessed on January 2, 2021) using blastp (version 2.11.0+) to see if they have an existing, high-similarity human sequence analog:
if a midP has ≤15 amino acids:
   blastp -db nr -query midp_le15.fa -out midp_le15.xml -outfmt 5 -evalue 1 -task blastp-short -taxids 9606
else if a midP has <30 amino acids:
   blastp -db nr -query midp_lt30.fa -out midp_lt30.xml -outfmt 5 -evalue 1e-4 -task blastp-short -taxids 9606
otherwise:
   blastp -db nr -query midp_ge30.fa -out midp_ge30.xml -outfmt 5 -evalue 1e-4 -task blastp -taxids 9606

Here, "high-similarity" was defined as coverage ≥1 and aligned identity ≥0.9 for midPs with ≤15 amino acids, or coverage ≥0.9 and aligned identity ≥0.9 otherwise (coverage and aligned identity are parameters as above).

Similar procedures were performed for 10- to 14-nt matches, and the number of RNA hits, midRs, and midPs are summarized in Table 1.

**Table 1. Summary of the number of matched RNAs, midRs, and midPs derived from 15- to 10-nt miRNA-RNA reverse matches**

| Match length | Number of matched RNAs | Number of unique midRs¹ | Number of unique (and redundant) midPs² |
|---|---|---|---|
| 15-nt | 13,310 | 11,453 | 1,239 (5,369) |
| 14-nt | 29,079 | 24,492 | 2,135 (9,629) |
| 13-nt | 81,633 | 67,350 | 4,014 (22,829) |
| 12-nt | 246,696 | 201,067 | 4,252 (34,225) |
| 11-nt | 835,868 | 680,634 | 4,397 (71,208) |
| 10-nt | 2,953,351 | 2,401,247 | 3,784 (162,694) |

| | | | |
|---|---|---|---|
| ¹The number of redundant midRs is identical to the number of matched RNAs. ²Theoretically, it is expected that unique midPs derived from shorter matches will always cover those derived from longer matches, but this may not be true in practice because NCBI blast is a heuristic algorithm which means it may not report all alignments it finds and hence there is a chance that previous findings disappear as the database (of midRs) grows. | | | |

### Nomenclature of midR and midP identifiers and names

A nomenclature was developed to set identifiers for midRs and midPs that can reflect the nature that midRs and midPs are derived from the elongation of miRNA with RNA as template. The midR identifier is defined as "midR. {miRNA name}. {GenInfo Identifier number of the RNA (gene symbol of the RNA)}. {the end matching position on RNA}", in which "midR" is the prefix. In principle, we can similarly define midP identifiers by appending {start codon position on midR} to the midR identifier. A midP can be completely defined by template RNA together with the position on RNA that corresponds to the start codon on midR. Thus, a midP identifier is defined as "midP. {GenInfo Identifier number of the RNA (gene symbol of the RNA)}. {the midR start codon-paired position on RNA}". The midP identifiers generated in this way largely prevent using multiple midR-based midP identifiers for the same midP encoded by midRs derived from different miRNAs bound to one RNA.

Though meaningful, it should be noted that the above defined midR and midP identifier may still not be unique for the same midR or midP as a result of RNA variants caused by RNA splicing. Also, the identifiers are verbose for data presentation. Thus, alias names were also used to describe midRs and midPs in this work (see Tables 2 and 3).

**Table 2. Summary of four selected midPs for IHC assay**

| midP name | Length (aa) | Sequence | Immunogen sequence |
|---|---|---|---|
| midP0045 | 15 | MANIFPVFAPVTFRT (SEQ ID NO: 45) | CANIFPVFAPVTFRT (SEQ ID NO: 19713) |
| midP0112 | 25 | MDFSTKHPVFSLKHKCHHVTGSCLT (SEQ ID NO: 112 | PVFSLKHKCHHVT GSC (SEQ ID NO: 19714) |
| midP0188 | 171 |  (SEQ ID NO: 188) | LAPKPKPGGFQEQL RC (SEQ ID NO: 19715) |
| midP0323 | 68 |  (SEQ ID NO: 323) | CGMGHRAQPLRSD FKQ (SEQ ID NO: 19716) |

The predicted immunogenic peptide fragments in midPs are underlined, and they were further designed into peptide immunogens by adding a cysteine at the N terminus if the N-terminal or C-terminal amino acid is not cysteine.

**Table 3. Summary of the identifiers and names for selected midPs and corresponding midRs.**

| midP name | midP and midR Identifier(s)¹ | midR name 2 |
|---|---|---|
| midP0045 | midP.1370471854(RPS6KB1).414, midR.hsa-miR-1972.1370471854(RPS6KB1).2511 | midRs0045 |
| midP0112 | midP.1370451977(NAXE).1372, midR.hsa-miR-5585-3p.1370451977(NAXE).3203 | midRs0112 |
| midP0188 | midP.1677479768(AP1M1).3571, midR.hsa-miR-619-5p.1677479768(AP1M1).8997; midP.1677502062(AP1M1).3607, midR.hsa-miR-619-5p.1677502062(AP1M1).9033 | midRs0188 |
| midP0323 | midP.1819229406(C9orf129).1643, midR.hsa-miR-3686.1819229406(C9orf129).4888 | midRs0323 |

| | | |
|---|---|---|
| ¹Different midR sequences may encode the same midP sequence; such midRs can be derived from the elongation of the same miRNA bound to mRNA variants, or the elongation of different miRNAs bound to the same RNA. For instance, the two *AP1M1* variants 1 and 2 have 12,895 and 12,859 nucleotides, respectively; their only difference is that the 36-nt exon 6 in variant 1 is sliced in variant 2, resulting in a 36-nt shift of the follow-up exons in variant 2 (FIG. 6). For each miRNA able to bind to variant 1 at a position after exon 6, it can also bind to variant 2 at a corresponding position; thus, two *AP1M1* variants can yield similar midRs which may encode the same protein with different identifiers by the nomenclature (e.g., midP.1677479768(AP1M1).3571 and midP.1677502062(AP1M1).3607 are identifiers for midP0188). ²The numbers in the midR names were set the same as that in the midP names. | | |

### Cell line and transfection

Lung cancer cell line A549 and breast cancer cell line MDA-MB-231 were purchased from Cell Resource Center of Chinese Academy of Medical and authenticated by short tandem repeat analysis. All cell lines tested negative for mycoplasma. A549 cells were cultured in minimum Eagle's medium with 10% heat-inactivated fetal calf serum (Gibco, USA) and Penicillin-Streptomycin (Beyotime, China). MDA-MB-231 cells were cultured in Dulbecco's modified Eagle's medium with 10% heat-inactivated fetal calf serum (Gibco, USA) and penicillin-streptomycin (Beyotime, China). Cells were maintained at 37 °C with 5% CO₂.

For midP0188 knockdown, shRNA lentivirus was synthesized by Obio Technology (Shanghai, China). RNA interference oligonucleotides were annealed and inserted into the pSLenti-U6- shRNA-CMV-EGFP-F2A-Puro-WPRE lentiviral vector. The shRNA sequences are: shRNA-1, GGAGTCATCTCCACTGCATTT (SEQ ID NO: 19,717); shRNA-2, GCGTCTACTTCCTTCGTCTTG (SEQ ID NO: 19,718); shRNA-3, GCTAGGTGCGTCTGCTTCATT (SEQ ID NO: 19,719); and ctrl-shRNA, CCTAAGGTTAAGTCGCCCTCG (SEQ ID NO: 19,720).

### Transwell assay

Transwell chambers were placed into a 24-well plate and 300 µl pre-warmed serum-free medium was added to the upper chamber. The culture was discarded after 15 min at room temperature. The treated cells were trypsinized into single cells and resuspended in serum-free medium. After counting, the cells were seeded into the upper chamber of at 105 cells/well. 500 µl 10% fetal bovine serum medium was added to the lower chamber. All cells were cultured in an incubator with CO₂ at 37 °C for 24 h. After wiping cells in the upper chamber, 4% formaldehyde was used to fix cells for 30 min. After washing with phosphate buffered saline (PBS) and staining with 0.1% crystal violet, invaded cells were observed under a microscope (200× magnification).

### Wound healing assay

Cells were digested and seeded in 24-well plates at a density of 2×10⁵ cells/well. After cell density reached 90%, a 100 µl pipette tip was used to make straight scratches. The culture medium was discarded, and the cells were washed three times with PBS to remove cell debris and cultured with serum-free medium in an incubator with 5% CO₂ at 37°C. After 48 h, photos were taken and the cell migration rates were calculated.

### Scanning electron microscope imaging

Cells were first fixed in pre-chilled 2.5% glutaraldehyde at 4 °C for 2 h, and then dehydrated with a gradually increased concentration of ethanol (30%, 50%, 70%, 80%, 90%, 95%, and 100% v/v) for 10 min at each concentration. Following this, cells were treated with a gradually increased concentration of tert-butanol (30%, 50%, 70%, 80%, 90%, 95%, and 100% v/v) for 15 min at each concentration. Finally, cell phenotypes were observed under a scanning electron microscope (JEOL, Japan) after dried and gold spray.

### RT-PCR

Total cell RNAs were extracted using trizol (Beyotime, China) and quantified. 3 µg RNA was used to carry out reverse transcription (RT) reaction to obtain cDNA follow the instruction of PrimeScript RT Reagent Kit (Takara, Japan). RT primers are specific to midRs. Next, primers were designed that span at least two exons to run PCR with the cDNA as a template. The PCR products were identified by agarose gel electrophoresis and DNA sequencing. All primer sequences are provided in Table 4.

**Table 4. Summary of the PCR-amplified regions of selected midRs.**

| midR name¹ | Amplified region² | RT primer³ | Forward Primer³ | Reverse Primer³ |
|---|---|---|---|---|
| midRs0045 | 442-831 (exon 4-8) | ATGGCGGCAGCG GCTGTGGT (SEQ ID NO: 19721) | AAACTCCACCAAT CCACAGC (SEQ ID NO: 19722) | AGTAAAGCATCC CTTCATCG (SEQ ID NO: 19723) |
| midRs0112 | 186-675 (exon 1-5) | ATGGCGAGCACG GTGGTGAAG (SEQ ID NO: 19724) | TGGCAATGGGCA CAGTGAGT (SEQ ID NO: 19725) | GCGAGCACGGTG GTGAAGTA (SEQ ID NO: 19726) |
| midRs0188.1 | 602-1318 (exon 5-11) | GGTTGCCACATC CAAGAAGA (SEQ ID NO: 19727) | CCGGAGGTAGTG AAGTAAGG (SEQ ID NO: 19728) | AGGCATCAAGTA TCGGAAGA (SEQ ID NO: 19729) |
| midRs0188.2 | 594-969 (exon 5-9) | AGACCACCGAC AGCAAGAT (SEQ ID NO: 19730) | CGTGGGTGTTGA GACGGTAGG (SEQ ID NO: 19731) | CGGTCCGAAGGC ATCAAGTA (SEQ ID NO: 19732) |
| ssmidRs0323 | 156-577 (exon 1-3) | GCATCTTCCAGA TCCCAAT (SEQ ID NO: 19733) | CGATGTAGAAGA GCCAGGGAAGTA (SEQ ID NO: 19734) | TGGGATGTAACA GAGCAAGCG (SEQ ID NO: 19735) |

| | | | | |
|---|---|---|---|---|
| ¹Two regions were amplified for midRs0188, namely midRs0188.1 and midRs0188.2. ² Amplified regions are indexed on template RNAs. ³ All primers are from 5' to 3'. | | | | |

### Western blot

Cell proteins were extracted using radio immunoprecipitation assay lysis buffer that contains protease inhibitors (Beyotime, China). After quantification using BCA Protein Assay Kit (Beyotime, China), proteins were separated by 10% SDS-PAGE and transferred to the PVDF membrane (Millipore, USA). The membrane was placed in 5% bovine serum albumin and incubated with the midP0188 antibody (1:500 dilution) or the actin antibody (1:2000, Affinity, USA) for 2 h at room temperature, and then incubated with horseradish peroxidase-labeled secondary antibody (1:3000, Affinity, USA) at room temperature for 1 h. After washing the membrane with phosphate buffered saline with TWEEN20 for three times, we used the ECL Chemiluminescence Kit (Beyotime, China) to detect proteins.

### Silver staining

After the cells were washed twice with 1 × PBS, proteins were extracted using 500 µl cell lysate (P1048, Beyotime, China). 50 µl protein A/G magnetic beads (HY-K0202, MCE, USA) were incubated with midP0188 antibody at room temperature for 30 min, and then the harvested beads were incubated with protein at 4 °C for 2 h. The protein complex eluted using 1 × SDS-PAGE loading buffer was then separated by SDS-PAGE electrophoresis. The silver staining was performed according to instructions (P0017S, Beyotime, China). The gel was fixed with 100 ml fixative solution at room temperature for 20 min, then rinsed with 30% ethanol and pure water for 10 min. Silver solution was then treated for 10 min at room temperature and rinsed with pure water. Then, the reaction was terminated after being treated in the color developing solution for 10 min.

### Mass spectrometry

Acuity UPLC (Thermo Scientific) connected to a QExactive orbitrap (Thermo Scientific) was used for protein identification using the SDS-PAGE bands by peptide mapping. The bands of interest were cut out of the gel, de-stained, and subjected to an in-situ trypsin digestion for 16 h at 37 °C. The digested peptides were extracted by solution (acetonitrile-formic acid-water 7:1:2, v/v/v) and directly dried by vacuum concentrator. The peptides were separated using an UPLC system (Thermo Scientific) equipped with a reversed phase CSH C18 column (150 mm, 1.7 µm, Waters) and analyzed using a QExactive orbitrap (Thermo Scientific) run in highsensitivity mode. The flow rate was 200 µl/min and the column temperature was kept at 40 °C. Solvent A consisted of 0.1% formic acid (FA) in water. Solvent B consisted of 0.1% FA in acetonitrile. During the 120-min gradient the solvent B level was increased from 2% to 29 % in 60 min, from 29% to 55 % in 5 mins, from 55% to 75% in 2 min and remained at 75% for 5 min, and then returned to 2% over 2 min and stayed at 2% for the remaining 6 min. The masses of the digested peptides were then entered into the NCBI nr database (Human) using the Sequest search engine (Thermo) to identify the proteins. The digestion products by Glu-C and chymotrypsin were similarly analyzed.

### CSSD synthesis

CSSD4 was designed that can absorb four miRNAs and a control CSSD (Ctrl-CSSD) with scramble DNA. For Ctrl-CSSD, the single-stranded DNA (5'-AATTCAAAGAATTAACCTTAATTGAA GGGGAGGGTTCAGTACTTTTGTGTAGTACAAATATCAGTACTTTTGTGTAGTACAAA AGGGAGGGCTTCAATTAAGGTTAATTCTTTG-3' (SEQ ID NO: 19736) was treated with T4 DNA ligase (Takara, Beijing, China) after annealing. For CSSD4, two single-stranded DNAs were synthesized: one contains miR-619-5p and miR-3135b binding sequences (5'-GAATTCAAAGAATTAACCTTAATTGAAGGGGAGGGTTCACCACTGCACTCGCTCCAG CCATATGGCTCATGCCTGTAATCCCAGCAAGGGAGGGCTTCAATTAAGGTTAATTCT TTG -3' (SEQ ID NO: 19737)); the other contains miR-4728-3p and miR-5585-3p binding sequence (5'-AATTCAAAGAATTAACCTTAATTGAAGGGGAGGGTTCTGGGGCAGGAGGGAGGTCA GCATGATATACCTGTAGTCCCAGCTATTCAGAAGGGAGGGCTTCAATTAAGGTTAAT TCTTT-3' (SEQ ID NO: 19738)). After annealing, the two DNA strands were treated with T4 DNA ligase to form a completely closed circular DNA (FIG. 7). All single-stranded DNAs were synthesized by Genewiz (Beijing, China).

### Antibody

For chosen midPs, specific peptide immunogens were designed and synthesized (Apeptide, Shanghai, China). After mass spectrometry verification, the synthetic peptides were cross-linked to the Keyhole limpet hemocyanin protein to prepare immunogenic antigens. For the first immunization, 350 µg antigen and 1000 µl Freund's complete adjuvant (Sigma, F5881) were mixed and injected into the rabbits' skin. For 3 follow-up immunizations, 200 µg antigen mixed with 1000 µl Freund's incomplete adjuvant (Sigma, F5506) was injected each time. After the rabbits were euthanized, blood was collected and centrifuged to obtain serum for subsequent immunoaffinity purification. After cross-linking peptides and epoxy modified agarose (Wechsler Chromatography, Beijing), the serum was combined with peptides and eluted to obtain affinitypurified specific antibodies, which were then used for western blot and immunohistochemical staining assays. All immunogenic peptide sequences are provided in Table 3.

### Clinical specimens

Paraffin-embedded tumor samples from Qinhuangdao Municipal No. 1 Hospital were reviewed by pathologist. The study was performed in accordance with the guidelines from the declaration of Helsinki and its amendments or comparable ethical standards.

### Immunohistochemistry (IHC) assay

Tumor tissues were fixed with 10% formalin and embedded in paraffin. For IHC, the embedded tissues were cut into 4 µm-thick sections. The sodium citrate solution (Beyotime, China) was used for antigen retrieval, followed by blocking with 3% H₂O₂. Then the sections were incubated with midP0188 antibody (1:200) overnight at 4 °C in the dark. After washing with PBS and incubated with horseradish peroxidase-conjugated secondary antibody (1:300, Affinity, USA), the DAB kit (Beyotime, China) and hematoxylin were used for staining development and counterstaining, respectively.

### In vivo study

BALB/C nude mice were used for evaluating tumor growth and metastasis. For the tumor growth experiments, 24 mice were randomly divided into four groups. Two groups were subcutaneously inoculated with A549 cells at a concentration of 5 × 106 cells/mouse, and were then treated with CSSD4 or Ctrl-CSSD through intratumoral injection at 20 µg/mouse once every seven days for a total of three times. The same procedures were conducted on the other two groups with MDA-MB-231 cells used. All mice were euthanized on day 34 by CO₂ asphyxiation. For the tumor metastasis experiments, 12 mice were randomly divided into four groups. Two groups of mice were inoculated with A549 cells into the tail veins at 5 × 10⁶ cells/mouse, and ten days later CSSD4 or Ctrl-CSSD was injected into the tail veins for treatment at 30 µg/mouse once every seven days for three times. All animal experiments were permitted and carried out according to the practices prescribed by the Institutional Animal Care and Use Committee (IACUC) of the Tianjin International Joint Academy of Biomedicine.

### Statistical analysis

Statistical analyses were performed using GraphPad Prism software 9.0 (La Jolla, CA, USA), and the results were expressed as the mean ± standard deviation. Comparisons between two groups were performed using unpaired, two-tailed student's t-test. Comparisons between multiple groups were performed using one-way ANOVA and Tukey's post-hoc test. Significance is shown as: **, P < 0.01; *, P < 0.05.

### Data availability

The mature miRNA sequences were downloaded from the miRbase website (https://www.mirbase.org/ftp.shtml, accessed on December 20, 2020). The RefSeq_RNA database was downloaded from (https://ftp.ncbi.nlm.nih.gov/blast/db/, accessed on December 20, 2020). The experimental IRES sequences were downloaded from the IRESbase website (http://reprod.njmu.edu.cn/cgi-bin/iresbase/index.php, accessed on January 2, 2021). The NCBI nr database was downloaded from (https://ftp.ncbi.nlm.nih.gov/blast/db/, accessed on January 2, 2021).

### Results

Four midPs suggested by the MS data were selected to verify the expression levels of their encoding midRs in A549 cells (Table 3). For each midR(s), primers were designed to amplify the sequences that only exist in the complementary DNA (cDNA) sequences of the midR but not in the human genomic DNA sequences (FIG. 1B, Table 4); two fragments were amplified for midRs0188, namely midRs0188.1 and midRs0118.2, as dual verification (FIG. 1C, Table 4). Reverse transcript (RT)-PCR and follow-up Sanger sequencing results confirmed the existence of these sequences, showing the presence of midRs that encode midP0112 and midP0188 (FIG. 1C). The sequences for the other two midRs, midR0045 and midR0323, were not detected in these cells (FIG. 1C).

The existence of the two midPs, miP0112 and midP0188, was determined next. Their amino acid sequences were analyzed, peptide immunogens were designed, and rabbit polyclonal antibodies (pAbs) were derived for each of these midPs (Table 2). The anti-midP pAbs were used for immunohistochemistry (IHC) assays to detect the expression of midP0112 and midP0188 in human cancer and normal (control) tissues. The anti-midP0188 pAb induced strong, positive staining signals in lung and breast cancer tissues (Fig. 9), whereas the anti-midP0112 pAb resulted in positive signals in the tissues from lung and colon cancers (Fig. 10). The results show that both pABs stained positive in the tissue sections, indicating the expression of these midPs in human cancer tissues.

The existence of midP0188 was further confirmed, because the IHC staining data showed that the intensity of the midP0188 signals in both the lung and breast cancer tissues are much higher than those in the normal lung and breast tissues (FIG. 2) from different patients.

First, the anti-midP0188 pAb was incubated with the lung tumor cell line A549 lysates, followed by target protein (midP0188) capture with the protein A/G magnetic beads. SDS-PAGE was run to separate the lysate, and an 18 kDa band was observed, matching the predicted molecular weight of midP0188 (FIG. 1D).

The 18 kDa band was harvested, digested using trypsin, chymotrypsin, or Glu-C (staphylococcal V8 protease) separately, followed by mass spectrometry analysis. In total, 13 peptide fragments appearing in midP0188 were confirmed, of which 3 were from trypsin digestion, 3 were from Glu-C digestion, and 7 were from chymotrypsin digestion (FIG. 1E). The cleavage positions are all consistent with the canonical digestive sites of three proteases. These 13 fragments cover a total of 55% amino acids (94/171) in the midP0188 sequence (FIG. 1E). When we subjected the sequences of the four longest consecutive peptide fragments against the nr database, no identical or high-similarity entries were found, again, confirming that these fragments are previously unknown biological entities.

Next, we worked to test if downregulating the midP0188's originating miRNAs would knockdown this protein. MidP0188 was predicted by using the stringent screening threshold of 15-nt match between a miRNA's 3' end and an RNA. Specifically, hsa-miR-619-5p is the miRNA that was predicted to bind mRNA AP1M1 variant 1 or variant 2, which both give rise to midRs0188 that encode midP0188. We note however, if we relax the matching threshold (e.g., from 15-nt to 14-nt, 13-nt, 12-nt, or fewer), there may be additional miRNAs that could give rise to midP0188-encoding midRs (FIG. 6). Therefore, to knockdown midP0188, targeting all or the most potential originating miRNAs is optimal.

We hence relaxed the matching number to 12-nt and identified four miRNAs that can give rise to midP0188. These four miRNAs are hsa-miR-619-5p, hsa-miR-3135b, hsa-miR-4728-3p, and hsa-miR-5585-3p, all making a ≥12-nt match with AP1M1 (FIG. 6).

We designed a circular single-stranded DNA (CSSD) to knockdown all these four miRNAs simultaneously, which was named CSSD4 (FIG. 1F and FIG. 7). We previously reported the development of CSSD as an adsorbing molecule for miRNAs as an efficient knockdown method (12). Transfection of CSSD4 to A549 cells effectively reduces the levels of all the four miRNAs (FIG. 1G). Consistently, the levels of midP0188 in the CSSD4-treated MDA-MB-231 and A549 tumor cells are markedly reduced (FIG. 1H).

After confirming that targeting the originating miRNAs effectively knock down miP0188, we moved down the axis to ask if targeting the midRs would also similarly reduces the miP0188 protein levels. To achieve this, we generated stable cell lines expression short hairpin RNAs (shRNA) targeting midP0188 coding midRs, in both MDA-MB-231 and A549 cells. Marked reduction of midP0188 protein levels were observed (FIG. 8).

MidPs and their corresponding midRs represent a brand-new cohort of biological identities. It is contemplated that many midPs and midRs play important roles in human biology and disease. It is further contemplated that some midPs and midRs provide biomarkers and/or therapeutic targets for human diseases such as cancer. midP0188, and its originating miRNAs and its encoding midRs were further explored as targets for cancer drug development.

After the CSSD4 transfection in tumor cell lines MDA-MB-231 and A549 cells, which target four midP0188 originating miRNAs, midP0188 protein levels were markedly decreased (FIG. 1H). Transwell and wound healing assays show that CSSD4-treated cells had compromised invasion (FIG. 3B) and migration (FIG. 3C) capacities, compared to these two tumor line cells that received a vehicle control CSSD. Scanning electron microscopy imaging revealed that CSSD4 treatment adversely altered the morphology of these tumor cells (FIG. 3A).

Similar results were observed when we used a lentivirus to target the two encoding miRs for miP0188 in MDA-MB-231 and A549 cells. In comparison to the vehicle treated cells, lentivirus treated tumor cell had compromised invasion and migration capacity in the transwell and wound healing assays, and altered tumor cell morphology by scanning electron microscopy (FIG. 8).

Next, to examine the effects of targeting midP0188 on tumor growth and metastasis in vivo, we established subcutaneous solid tumor model and tail vein metastasis mouse models for lung and breast cancers.

In the subcutaneous solid tumor model, 24 five-week-old BALB/c nude mice were randomly divided into four groups. A549 or MDA-MB-231 cells were inoculated subcutaneously at a concentration of 5×10⁶ cells/mouse. When the tumor size reached 5 mm in diameter, CSSD4 or Ctrl-CSSD was injected intratumorally to the tumor mass. Ten days after cell injection, tumor sizes were measured every three days (FIG. 3E). After three administrations of CSSD4 or Ctrl-CSSD, the mice were euthanized, and solid tumor tissues were collected for size measurement and IHC analysis. The results showed that tumor sizes in the CSSD4 groups were significantly smaller than those in Ctrl-CSSD groups for both tumor cell types (FIG. 3D). At the same time, IHC staining revealed that the protein levels of midP0188 were effectively reduced (FIG. 3F).

In the tail vein metastasis model, 12 five-week-old BALB/c nude mice were randomly divided into four groups. MDA-MB-231 and A549 cells that stably express luciferase were injected into nude mice through the tail vein at a concentration of 5×10⁶ cells/mouse. Ten days later, CSSD4 or Ctrl-CSSD (30 µg/mouse) was administered intravenously. The metastasis of tumor cells in nude mice was evaluated after three administrations. The results indicated that the CSSD4-treated animals suffered much less lung metastasis than those in the control group (FIG. 3G).

These in vitro and in vivo experiment results reveal that midP0188 plays an important role in promoting lung and breast tumor genesis and metastasis, and downregulating midP0188 represents a strategy to treat lung and breast cancers.

The invention can also be defined by the following clauses:
1. An isolated miRNA-derived RNA (midR).
2. A composition comprising an isolated miRNA-derived RNA (midR).
3. A composition consisting essentially of an isolated miRNA-derived RNA (midR).
4. The midR or composition of any of clauses 1-3, wherein said midR comprises a sequence selected from the group consisting of SEQ ID NO: 8260-19712.
5. The midR or composition of any of clause 1-3, wherein said midR consists of a sequence selected from the group consisting of SEQ ID NO: 8260-19712.
6. The midR or composition of any of clauses 1-3, wherein said midR comprises a sequence having at least 70% identity to a sequence selected from the group consisting of SEQ ID NO: 8260-19712.
7. The midR or composition of any of clauses 1-3, wherein said midR comprises a sequence having at least 80% identity to a sequence selected from the group consisting of SEQ ID NO: 8260-19712.
8. The midR or composition of any of clauses 1-3, wherein said midR comprises a sequence having at least 90% identity to a sequence selected from the group consisting of SEQ ID NO: 8260-19712.
9. The midR or composition of any of clauses 1-3, wherein said midR comprises a sequence having at least 95% identity to a sequence selected from the group consisting of SEQ ID NO: 8260-19712.
10. The midR or composition of any of clauses 1-3, wherein said midR comprises a sequence having at least 99% identity to a sequence selected from the group consisting of SEQ ID NO: 8260-19712.
11. The midR or composition of any of clauses 1-10, wherein said midR encodes midP0188.
12. An isolated DNA comprising a sequence complementary to a midR of any of clauses 1 or 4-11.
13. An isolated DNA comprising a sequence complementary to at least 50 consecutive nucleotides of a midR of any of clauses 1 or 4-11.
14. A composition comprising the DNA of clause 12 or 13.
15. The midR, DNA, or composition of any of clauses 1-14, wherein said midR or DNA further comprises a label.
16. The midR, DNA, or composition of clause 15, wherein the label is a fluorescent label.
17. The midR, DNA, or composition of any of clauses 1-16, wherein said midR or DNA is directly or indirectly attached to a solid support.
18. The midR, DNA, or composition of any of clauses 1-17, wherein said midR or DNA further comprises an adapter.
19. The midR, DNA, or composition of any of clauses 1-18, wherein said midR or DNA further comprises a unique molecule identifier (UMI).
20. A composition comprising a midR or DNA of any of clauses 1, 4-13, or 14-19 and a primer complementary to a portion of said midR or DNA.
21. A composition comprising a midR or DNA of any of clauses 1, 4-13, or 14-19 and a probe complementary to a portion of said midR or DNA.
22. A composition comprising a midR or DNA of any of clauses 1, 4-13, or 14-19 fused to a nucleic acid fusion partner.
23. The composition of clause 22, wherein said fusion partner encodes a purification tag.
24. A composition comprising a vector comprising a midR or DNA of any of clauses 1 or 4-13.
25. The composition of clause 24, wherein said vector comprises an expression vector.
26. A cell comprising a heterologous midR or a heterologous regulatory sequence regulating expression of a midR.
27. A cell comprising DNA complementary to a midR.
28. The cell of clause 27, wherein said DNA is contained in a vector.
29. The cell of clause 27, wherein said DNA is expressible in said cell.
30. The cell of clause 26 or 27, wherein said midR is selected from the group consisting of SEQ ID NO: 8260-19712 or a variant thereof.
31. The cell of any of clauses 26- 30, wherein said midR encodes midP0188.
32. An isolated miRNA-derived peptide (midP).
33. A composition comprising an isolated miRNA-derived peptide (midP).
34. A composition consisting essentially of an isolated miRNA-derived peptide (midP).
35. The midP or composition of any of clauses 32-34, wherein said midP comprises a sequence selected from the group consisting of SEQ ID NO: 1-8259.
36. The midP or composition of any of clause 32-34, wherein said midP consists of a sequence selected from the group consisting of SEQ ID NO: 1-8259.
37. The midP or composition of any of clauses 32-34, wherein said midP comprises a sequence having at least 70% identity to a sequence selected from the group consisting of SEQ ID NO: 1-8259.
38. The midP or composition of any of clauses 32-34, wherein said midP comprises a sequence having at least 80% identity to a sequence selected from the group consisting of SEQ ID NO: 1-8259.
39. The midP or composition of any of clauses 32-34, wherein said midP comprises a sequence having at least 90% identity to a sequence selected from the group consisting of SEQ ID NO: 1-8259.
40. The midP or composition of any of clauses 32-34, wherein said midP comprises a sequence having at least 95% identity to a sequence selected from the group consisting of SEQ ID NO: 1-8259.
41. The midP or composition of any of clauses 32-40, wherein said midP comprises midP0188.
42. The midP or composition of any of clauses 32-41, wherein said midP further comprises a label.
43. The midP or composition of clause 42, wherein the label is a fluorescent label.
44. The midP or composition of any of clauses 32-43, wherein said midP is directly or indirectly attached to a solid support.
45. A composition comprising a midP of any of clauses 32 or 35-44 and a binding molecule that specifically binds to said midP.
46. The composition of clause 45, wherein said binding molecule comprises an antibody.
47. A composition comprising a midP of any of clauses 32 or 35-44 fused to a fusion partner.
48. The composition of clause 47, wherein said fusion partner is a purification tag.
49. A cell comprising a heterologous midP.
50. A cell comprising an expression vector encoding a midP.
51. The cell of clause 49 or 50, wherein said midP is selected from the group consisting of SEQ ID NO: 1-8259, or a variant thereof.
52. The cell of clause 49 or 50, wherein said midP is midP0188.
53. An antibody that specifically binds to a midP of any of clauses 32 or 35-41.
54. An array comprising a solid support comprising two or more sequences complementary two or more midR of any of clauses 1 or 4-11.
55. The array of clause 54, comprising 100 or more of said sequences.
56. An array comprising a solid support directly or indirectly attached to two or more midR of any of clauses 1 or 4-11.
57. The array of clause 56, comprising 100 or more said midR.
58. A method, comprising: identifying the presence of one or more midR in a sample.
59. The method of clause 58, wherein said one or more midR are selected from the group consisting of SEQ ID Nos: 8260-19712.
60. The method of clause 58, wherein said one or more midR comprises a midR that encodes for midP0188.
61. The method of clause 58, wherein said sample is tissue sample.
62. The method of clause 58, wherein said sample is a biological fluid sample.
63. The method of clause 58, wherein said sample is a cancer cell or derived from a cancer cell.
64. A method, comprising: identifying the presence of one or more midP in a sample.
65. The method of clause 64, wherein said one or more midP are selected from the group consisting of SEQ ID Nos: 1-8259.
66. The method of clause 64, wherein said one or more midP comprises midP0188.
67. The method of clause 64, wherein said sample is tissue sample.
68. The method of clause 64, wherein said sample is a biological fluid sample.
69. The method of clause 64, wherein said sample is a cancer cell or derived from a cancer cell.
70. A method comprising: depleting a cell of one or more midR or midP.
71. The method of clause 70, wherein said cell resides in vitro.
72. The method of clause 70, wherein said cell resides in vivo.
73. The method of clause 70, wherein said method comprises introducing into said cell one or more nucleic acid sequences that bind to one or more miRNAs that generates said midRs or midPs.
74. The method of any one of clauses 70 to 74, wherein said one or more midP comprises midP0188.
75. A method comprising: contacting a cell with a midR- or midP-specific inhibitor or agonist.
76. The method of clause 75, wherein said inhibitor is an antisense oligonucleotide that binds to a midR.
77. The method of clause 75, wherein said inhibitor is an antisense oligonucleotide that binds to an mRNA and prevents generation of a midR.
78. The method of clause 75, wherein said inhibitor is a sequence editing system that alters the sequence of a midR.
79. The method of clause 75, wherein said inhibitor is a sequence editing system that alters the sequence of an mRNA encoding a midR such that a functional midR is not generated from the mRNA.
80. The method of clauses 78 or 79, wherein said sequence editing system comprises a CRISPR/Cas system.
81. The method of any one of clauses 75 to 80, wherein said midR encodes midP0188.
82. The method of clause 75, wherein said inhibitor specifically binds to a midP.
83. The method of clause 82, wherein said midP is midP0188.
84. The method of clause 82, wherein said inhibitor is an antibody or antibody fragment.
85. The method of clause 82, wherein said inhibitor is an aptamer.
86. The method of clause 75, wherein said inhibitor prevents the generation of an miRNA.
87. The method of clause 86, wherein said inhibitor prevents the processing of a primiRNA.
88. A non-human animal expressing a heterologous midR.
89. The animal of clause 88, wherein said midR is a human midR.
90. The animal of clause 88 or clause 89, wherein said midR encodes midP0188.
91. A non-human animal comprising a heterologous midP.
92. The animal of clause 91, wherein said midP is a human midP.
93. The animal of clause 91 or 92, wherein said midP is midP0188.
94. The cell of any of clauses 26-31 or 49-52 wherein said cell is in culture.
95. The cell of clause 94, wherein said cell is a cancer cell.
96. A composition comprising a midP or an immunogenic fragment thereof and an adjuvant.
97. The composition of clause 96, wherein the midP is selected from the group consisting of SEQ ID NO: 1-8259.
98. The composition of clause 96, wherein the midP is midP0188.
99. A method of inducing an immune response, comprising: exposing an animal to an exogenous midP or an immunogenic portion thereof.
100. The method of clause 99, wherein said exogenous midP is a synthetic midP.
101. The method of clause 99 or 100, wherein said animal is further exposed to an adjuvant.
102. The method of any one of clauses 99 to 101, wherein the midP is midP0188.
103. A method of identifying a midR or midP, comprising: a) aligning an miRNA sequence to one or more RNA sequences; b) determining a sequence of a templated extension product of said miRNA; and c) selecting extension products that contain a start codon within 100 bases of an internal ribosome entry site (IRES).
104. The method of clause 103, wherein one or more or all of said aligning, determining, and selecting steps are conducted in silico.
105. The method of clause 103 or 104, further comprising the step of synthesizing a selected midR or midP.
106. The method of clause 103 or 104, further comprising the step of purifying a selected midR or midP from a sample.
107. The method of any of clauses 103-106, further comprising the step of immunizing an animal with a selected midP or an immunogenic fragment thereof.
108. The method of clause 107, further comprising the step of purifying an antibody generated in said animal.
109. The method of clause 108, further comprising the step of contacting a sample with said antibody to identify the presence of said midP.
110. The method of any one of clauses 103 to 109, wherein said midP is midP0188.
111. The method of any of clause 103 or 104, further comprising the step of synthesizing a primer or probe oligonucleotide that is complementary to a portion of said midR.
112. The method of clause 111, further comprising the step of contacting a sample with said primer or probe to identify the presence of said midR.
113. The method of 106, further comprising the step of sequencing said selected midR.
114. A non-transitory computer-readable medium storing a program configured to carrying out the method of clause 103.
115. A non-transitory computer-readable medium comprising an electronic representation of one or more of SEQ ID NOs:1-19712.
116. A method of sequencing at least one midR comprising: a) contacting a sample comprising one or more midRs with adapters such that one or more midR-adapter sequences are generated; and b) subjecting said midR-adapter sequences to a sequencing protocol such that at least one midR sequence read is generated.
117. The method of clause 116, further comprising: c) aligning said at least one midR sequence read with a reference midR sequence known to be cancer related.
118. The method of clause 116 or clause 117, wherein said adapters comprise at least one of the following: a universal primer binding sequence, a grafting sequence, a unique barcode, and a sample-specific index sequence.
119. A method for diagnosing a disease or condition in a subject, the method comprising the steps of: a) determining the levels of a midR or midP in a sample from the subject; and b) comparing the determined levels of the midR or midP to a reference value, wherein an increase or decrease in the levels of the midR or midP in the sample is indicative of the subject having the disease or condition.
120. The method of clause 119, wherein said midP is midP0188 and the disease or condition is cancer.
121. A method for monitoring a disease or condition in a subject, the method comprising the steps of: a) determining the levels of an midR or midP in a first sample from the subject; and b) determining the levels of the midR or midP in a second sample from the subject, wherein the second sample has been obtained from the subject at a later time point than the first sample, wherein: i) an increase or decrease in the levels of the midR or midP in the second sample as compared to the first sample is indicative of the disease or condition progressing or regressing in the subject.
122. The method of clause 121, wherein the midP is midP0188 and the disease or condition is cancer.
123. A method for monitoring a subject's compliance to a prescribed treatment for a disease or condition, the method comprising the steps of: a) determining the levels of a midR or midP in a first sample from the subject; and b) determining the levels of the midR or midP in a second sample from the subject, wherein the second sample has been obtained from the subject after the prescribed treatment, wherein an increase or decrease or no change in the levels of the midR or midP in the second sample as compared to the first sample is indicative of a subject's compliance to the prescribed treatment for the disease or condition.
124. The method of clause 123, wherein said midP is midP0188 and the disease or condition is cancer.
125. A method of monitoring therapeutic effect of a prescribed treatment for a disease or condition, the method comprising the steps of: a) determining the levels of an midR or midP in a first sample from the subject; and b) determining the levels of the midR or midP in a second sample from the subject, wherein the second sample has been obtained from the subject after the prescribed treatment, wherein a change or no change in the levels of the midR or midP in the second sample in the second sample as compared to the first sample is indicative of a therapeutic effect of a prescribed treatment for the disease or condition.
126. The method of clause 125, wherein said midP is midP0188 and the disease or condition is cancer.

### References

1. T. X. Lu, M. E. Rothenberg, MicroRNA. J Allergy Clin Immunol 141, 1202-1207 (2018).
2. M. Chalfie, H. R. Horvitz, J. E. Sulston, Mutations that lead to reiterations in the cell lineages of C. elegans. Cell 24, 59-69 (1981).
3. V. Ambros, H. R. Horvitz, Heterochronic mutants of the nematode Caenorhabditis elegans. Science 226, 409-416 (1984).
4. G. P. Brennan, D. C. Henshall, MicroRNAs as regulators of brain function and targets for treatment of epilepsy. Nat Rev Neurol 16, 506-519 (2020).
5. T. Kim, C. M. Croce, MicroRNA and ER stress in cancer. Semin Cancer Biol 75, 3-14 (2021).
6. T. J. Lee et al., Strategies to Modulate MicroRNA Functions for the Treatment of Cancer or Organ Injury. Pharmacol Rev 72, 639-667 (2020).
7. Y Tay, J. Zhang, A. M. Thomson, B. Lim, I. Rigoutsos, MicroRNAs to Nanog, Oct4 and Sox2 coding regions modulate embryonic stem cell differentiation. Nature 455, 1124-1128 (2008).
8. S. Vasudevan, Y Tong, J. A. Steitz, Switching from repression to activation: microRNAs can up-regulate translation. Science 318, 1931-1934 (2007).
9. U. A. Orom, F. C. Nielsen, A. H. Lund, MicroRNA-10a binds the 5'UTR of ribosomal protein mRNAs and enhances their translation. Mol Cell 30, 460-471 (2008).
10. Y Maida et al., An RNA-dependent RNA polymerase formed by TERT and the RMRP RNA. Nature 461, 230-235 (2009).
11. P. Weber et al., Therapy-related transcriptional subtypes in matched primary and recurrent head and neck cancer. Clin Cancer Res, (2021).
12. J. Meng et al., Derepression of co-silenced tumor suppressor genes by nanoparticle-loaded circular ssDNA reduces tumor malignancy. Sci TranslMed 10, (2018).
13. Y Gao et al., Structure of the RNA-dependent RNA polymerase from COVID-19 virus. Science 368, 779-782 (2020).
14. B. Malone, N. Urakova, E. J. Snijder, E. A. Campbell, Structures and functions of coronavirus replication-transcription complexes and their relevance for SARS-CoV-2 drug design. Nat Rev Mol Cell Biol 23, 21-39 (2022).
15. T. Krischuns, M. Lukarska, N. Naffakh, S. Cusack, Influenza Virus RNA-Dependent RNA Polymerase and the Host Transcriptional Apparatus. Annu Rev Biochem 90, 321-348 (2021).
16. Y Maida, M. Yasukawa, K. Masutomi, De Novo RNA Synthesis by RNA-Dependent RNA Polymerase Activity of Telomerase Reverse Transcriptase. Mol Cell Biol 36, 1248-1259 (2016).

## Claims

1. An isolated miRNA-derived RNA (midR).

2. A composition comprising or consisting essentially of an isolated miRNA-derived RNA (midR).

3. An isolated DNA comprising: a sequence complementary to, or at least 50 consecutive nucleotides of, a midR of claim 1.

4. A composition comprising the DNA of claim 3.

5. A composition comprising a midR or DNA of any of claims 1 or 3-4 and a primer or probe complementary to a portion of said midR or DNA.

6. A composition comprising a midR or DNA of any of claims 1 or 3-4 fused to a nucleic acid fusion partner.

7. A composition comprising a vector comprising a midR or DNA of claim 1 or claim 3.

8. A cell comprising a heterologous midR or a heterologous regulatory sequence regulating expression of a midR.

9. A cell comprising DNA complementary to a midR.

10. An isolated miRNA-derived peptide (midP).

11. A composition comprising or consisting essentially of an isolated miRNA-derived peptide (midP).

12. A composition comprising a midP of claim 10 and a binding molecule that specifically binds to said midP.

13. A composition comprising a midP of claim 10 fused to a fusion partner.

14. A cell comprising a heterologous midP or an expression vector encoding a midP.

15. An antibody that specifically binds to a midP of claim 10.

16. An array comprising a solid support comprising two or more sequences complementary two or more midR of claim 1.

17. An array comprising a solid support directly or indirectly attached to two or more midR of claim 1.

18. A method, comprising: identifying the presence of one or more midR in a sample.

19. A method, comprising: identifying the presence of one or more midP in a sample.

20. A method comprising: depleting a cell of one or more midR or midP.

21. A method comprising: contacting a cell with a midR- or midP-specific inhibitor or agonist.

22. A non-human animal expressing a heterologous midR.

23. A non-human animal comprising a heterologous midP.

24. A composition comprising a midP or an immunogenic fragment thereof and an adjuvant.

25. A method of inducing an immune response, comprising: exposing an animal to an exogenous midP or an immunogenic portion thereof.

26. A method of identifying a midR or midP, comprising: a) aligning an miRNA sequence to one or more RNA sequences; b) determining a sequence of a templated extension product of said miRNA; and c) selecting extension products that contain a start codon within 100 bases of an internal ribosome entry site (IRES).

27. A method of sequencing at least one midR comprising: a) contacting a sample comprising one or more midRs with adapters such that one or more midR-adapter sequences are generated; and b) subjecting said midR-adapter sequences to a sequencing protocol such that at least one midR sequence read is generated.

28. A method for diagnosing a disease or condition in a subject, the method comprising the steps of: a) determining the levels of a midR or midP in a sample from the subject; and b) comparing the determined levels of the midR or midP to a reference value, wherein an increase or decrease in the levels of the midR or midP in the sample is indicative of the subject having the disease or condition.

29. A method for monitoring a disease or condition in a subject, the method comprising the steps of: a) determining the levels of an midR or midP in a first sample from the subject; and b) determining the levels of the midR or midP in a second sample from the subject, wherein the second sample has been obtained from the subject at a later time point than the first sample, wherein: i) an increase or decrease in the levels of the midR or midP in the second sample as compared to the first sample is indicative of the disease or condition progressing or regressing in the subject.

30. A method for monitoring a subject's compliance to a prescribed treatment for a disease or condition, the method comprising the steps of: a) determining the levels of a midR or midP in a first sample from the subject; and b) determining the levels of the midR or midP in a second sample from the subject, wherein the second sample has been obtained from the subject after the prescribed treatment, wherein an increase or decrease or no change in the levels of the midR or midP in the second sample as compared to the first sample is indicative of a subject's compliance to the prescribed treatment for the disease or condition.

31. A method of monitoring therapeutic effect of a prescribed treatment for a disease or condition, the method comprising the steps of: a) determining the levels of an midR or midP in a first sample from the subject; and b) determining the levels of the midR or midP in a second sample from the subject, wherein the second sample has been obtained from the subject after the prescribed treatment, wherein a change or no change in the levels of the midR or midP in the second sample in the second sample as compared to the first sample is indicative of a therapeutic effect of a prescribed treatment for the disease or condition.

32. The midR, DNA, composition, cell, array, method or animal of any one of claims 1-14 or 16-31, wherein said midR comprises a sequence selected from the group consisting of SEQ ID NO: 8260-19712.

33. The midR, DNA, composition, cell, array, method or animal of any one of claims 1-14 or 16-31, wherein said midR encodes midP0188.

34. The midP, DNA, composition, cell, antibody, method or animal of any one of claims 1-15 or 18-31 wherein said midP comprises a sequence selected form the group consisting of SEQ ID NO: 188, 1-187 or 189-8259.

35. The midP, DNA, composition, cell, antibody, method or animal of any one of claims 1-15 or 18-31 wherein said midP comprises midP0188.

36. The method of any one of claims 25 to 35, wherein said midP is midP0188 and the disease or condition is cancer.

37. A non-transitory computer-readable medium storing a program configured to carrying out the method of claim 26.

38. A non-transitory computer-readable medium comprising an electronic representation of one or more of SEQ ID NOs: 1-19712.
